(19) Europäisches Patentamt
European Patent Office
Office européen des brevets

(11) EP 0 699 680 B1

(12) FASCICULE DE BREVET EUROPEEN

(45) Date de publication et mention de la délivrance du brevet: **08.03.2000 Bulletin 2000/10**

(51) Int Cl.[7]: **C07D 498/04**, A61K 31/435 // (C07D498/04, 263:00, 221:00)

(21) Numéro de dépôt: **95401989.9**

(22) Date de dépôt: **01.09.1995**

(54) **Dérivés d'oxazoloquinoleinone, leur préparation et leur application en thérapeutique**

Oxazolochinolinonderivate, ihre Herstellung und ihre Verwendung als Arzneimittel

Oxazoloquinolinone derivatives, their preparation and their use in therapy

(84) Etats contractants désignés:
**AT BE CH DE DK ES FR GB GR IE IT LI LU NL PT SE**
Etats d'extension désignés:
**LT LV SI**

(30) Priorité: **05.09.1994 FR 9410600**

(43) Date de publication de la demande:
**06.03.1996 Bulletin 1996/10**

(73) Titulaire: **Sanofi-Synthélabo
75013 Paris (FR)**

(72) Inventeurs:
- **Jegham, Samir
F-95100 Argenteuil (FR)**
- **Koenig, Jean Jacques
F-78600 Maisons Laffitte (FR)**
- **Puech, Frédéric
F-92500 Rueil Malmaison (FR)**
- **Burnier, Philippe
F-78600 Maisons Laffitte (FR)**
- **Zard, Lydia
F-91190 Gif sur Yvette (FR)**

(74) Mandataire: **Thouret-Lemaitre, Elisabeth et al
Sanofi-Synthélabo
Service Brevets
174, avenue de France
75013 Paris (FR)**

(56) Documents cités:
**EP-A- 0 322 263**

- **JOURNAL OF MEDICINAL CHEMISTRY., vol.11, no.6, 1968, WASHINGTON US pages 1161 - 1164 B. WILLIAM ET AL 'Central nervous system depressants. 2-Aminoalkyl-3,3a,4,5-tetrahy droimidazo(1,5-a)quinolin-1-(2H)-ones'**

Remarques:
Le dossier contient des informations techniques présentées postérieurement au dépôt de la demande et ne figurant pas dans le présent fascicule.

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

EP 0 699 680 B1

Printed by Jouve, 75001 PARIS (FR)

## Description

La présente invention a pour objet des dérivés de 3,3*a*,4,5-tétrahydro-1*H*-oxazolo[3,4-a]quinoléin-1-one, leur préparation et leur application en thérapeutique.
D'après le brevet européen EP 0 322 263, on connaît des dérivés de 3,3*a*,4,5-tétrahydro-1*H*-oxazolo[3,4-*a*] quinoléin-1-one, utiles comme agents antidépresseurs.
Les composés de l'invention répondent à la formule générale (I)

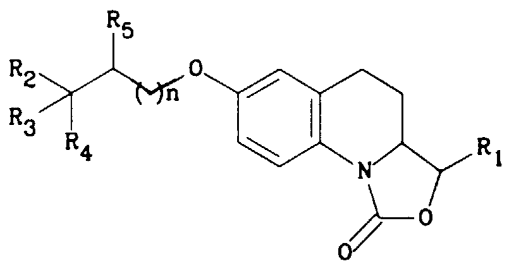

(I)

dans laquelle :

n est égal à 0 ou 1,
$R_1$ représente un atome d'hydrogène ou un groupe éthényle, méthyle, éthyle, phényle, hydroxyméthyle ou méthoxyméthyle, et, soit $R_2$ est un groupe méthyle, trifluorométhyle ou cyano, $R_3$ est un atome d'hydrogène ou un groupe hydroxyle ou benzyloxy et $R_4$ et $R_5$ sont des atomes d'hydrogène et si $R_2$ est un méthyle $R_3$ n'est pas un hydrogène,
soit $R_2$ et $R_4$ forment ensemble un groupe $-(CH_2)_4-$, $R_3$ est un groupe hydroxyle et $R_5$ est un atome d'hydrogène,
soit $R_2$ et $R_5$ forment ensemble un groupe $-O-(CH_2)_3-$, et $R_3$ et $R_4$ sont des atomes d'hydrogène,
soit $R_2$ et $R_5$ forment ensemble un groupe $-(CH_2)_4-$, $R_3$ est un groupe hydroxyle et $R_4$ est un atome d'hydrogène.

Les composés de l'invention peuvent exister sous forme d'énantiomères et de diastéréoisomères. La présente invention comprend ces différentes formes ainsi que leurs mélanges, y compris les mélanges racémiques.
Les composés de formule (I) peuvent être préparés selon le procédé représenté en annexe 1, qui consiste à traiter un composé de formule (II)

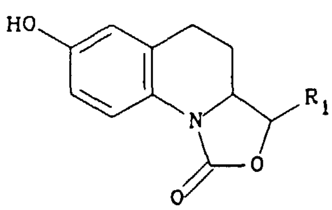

(II)

dans laquelle $R_1$ représente un atome d'hydrogène, un groupe éthényle, méthyle, phényle, hydroxyméthyle ou méthoxyméthyle, par un composé de formule (III)

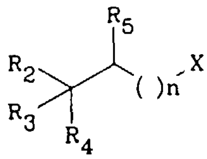

(III)

dans laquelle n, $R_2$, $R_3$, $R_4$ et $R_5$ sont définis comme dans la formule (I) et X représente un atome d'halogène ou un groupe labile tel que mésyloxy ou tosyloxy, pour obtenir le composé de formule (I) dans laquelle $R_1$ est défini comme précédemment, puis à réduire le composé de formule (I) dans laquelle $R_1$ est un groupe éthényle, pour obtenir le composé de formule (I) dans laquelle $R_1$ est un groupe éthyle.
Les composés de formule (II) dans laquelle $R_1$ représente un atome d'hydrogène, un groupe éthényle, méthyle

ou phényle, peuvent être préparés à partir du 2-formyl-6-méthoxy-1,2,3,4-tétrahydroquinoléine-1-carboxylate d'éthyle, composé connu dont la préparation est décrite dans le brevet EP 0 322 263.

Dans le cas où $R_1$ est un atome d'hydrogène, ce procédé consiste à traiter le 2-formyl-6-méthoxy-1,2,3,4-tétrahydroquinoléine-1-carboxylate d'éthyle par un agent réducteur tel que le borohydrure de sodium ou de potassium, à cycliser le composé obtenu, par réaction avec une base telle que le méthylate de sodium et enfin, à déméthyler la 7-méthoxy-3,3*a*,4,5-tétrahydro-1*H*-oxazolo[3,4-*a*]quinoléin-1-one de formule (IV)

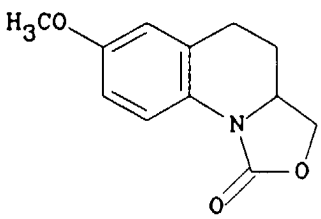

(IV)

Dans le cas où $R_1$ est un groupe éthényle, méthyle ou phényle, ce procédé consiste à traiter le 2-formyl-6-méthoxy-1,2,3,4-tétrahydroquinoléine-1-carboxylate d'éthyle par un organomagnésien de formule $R_1MgX$, dans laquelle $R_1$ représente un groupe éthényle, méthyle ou phényle et X un atome d'halogène, à cycliser le composé obtenu, par réaction avec une base telle que le méthylate de sodium et enfin, à déméthyler le dérivé de 7-méthoxy-3,3*a*,4,5-tétrahydro-1*H*-oxazolo[3,4-*a*]quinoléin-1-one de formule (V)

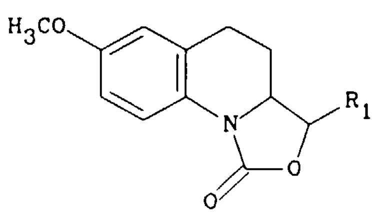

(V)

dans laquelle $R_1$ est défini comme précédemment.

Les composés de formule (II) dans laquelle $R_1$ représente un groupe hydroxyméthyle ou méthoxyméthyle peuvent être préparés à partir de la 3-éthényl-7-hydroxy-3,3*a*,4,5-tétrahydro-1*H*-oxazolo[3,4-*a*]quinoléin-1-one, composé de formule (II) dans laquelle $R_1$ représente un groupe éthényle, par protection du groupe hydroxyle pour obtenir un composé de formule (VI)

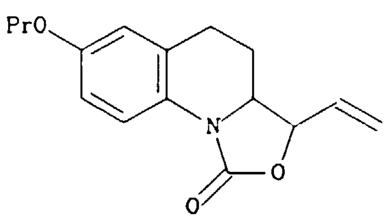

(VI)

dans laquelle Pr représente un groupe protecteur tel que benzyle,

qui est ensuite traité par l'ozone puis par un agent réducteur, tel que le borohydrure de sodium, pour donner un composé de formule (VII)

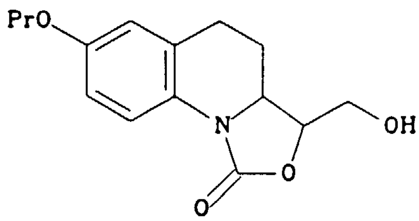

(VII)

qui est,

soit déprotégé pour donner un composé de formule (II) dans laquelle $R_1$ est un groupe hydroxyméthyle, soit traité par le sulfate de diméthyle pour donner un composé de formule (VIII)

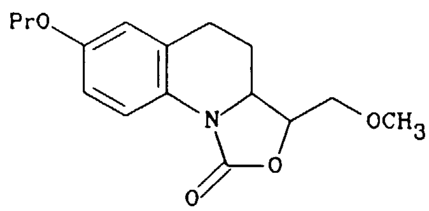

(VIII)

qui est ensuite déprotégé pour donner le composé de formule (II) dans laquelle $R_1$ représente un groupe méthoxyméthyle.

Les composés de formule (I) dans laquelle $R_1$ représente un groupe éthényle, méthyle, éthyle, hydroxyméthyle ou méthoxyméthyle existent sous forme d'isomères cis et trans

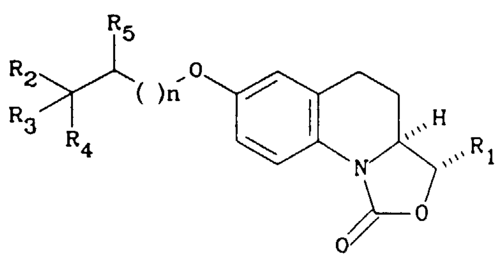

(I cis)

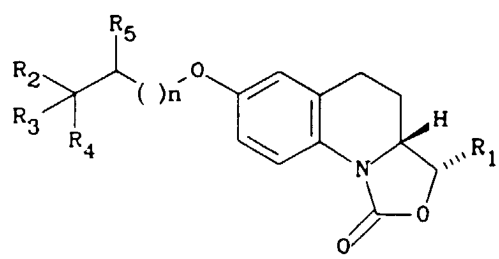

(I trans)

préparés respectivement à partir des isomères cis et trans des composés correspondants de formule (II), eux-mêmes obtenus selon le procédé tel que décrit ci-dessus, après séparation par chromatographie des isomères cis et trans du dérivé de formule (V)

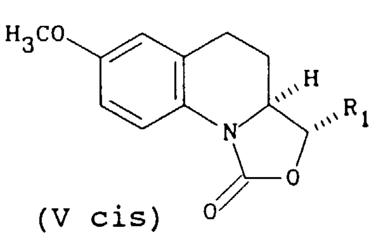

(V cis)

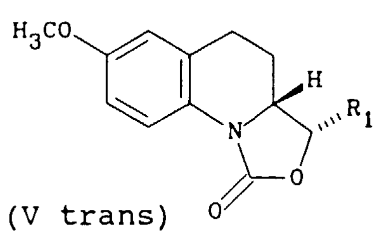

(V trans)

Les composés de formule (I) dans laquelle $R_1$ est un groupe éthényle et $R_3$ un groupe benzyloxy, ou bien $R_1$ est un groupe hydroxyméthyle ou méthoxyméthyle et $R_3$ un groupe hydroxyle ou benzyloxy, $R_4$ et $R_5$ sont des atomes

d'hydrogène et $R_2$ et n sont définis comme dans la formule générale (I), peuvent également être préparés selon le procédé représenté en annexe 2, qui consiste à traiter la 3-éthényl-7-hydroxy-3,3*a*,4,5-tétrahydro-1*H*-oxazolo[3,4-*a*] quinoléin-1-one, composé de formule (II) dans laquelle $R_1$ représente un groupe éthényle, par un composé de formule $R_2CH(OH)-(CH_2)-(CH_2)_nX$, dans laquelle $R_2$ et n sont définis comme ci-dessus et X est un atome d'halogène ou un groupe labile, tel que tosyloxy ou mésyloxy, à traiter le composé obtenu, de formule (IX)

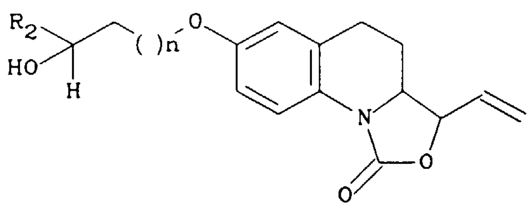

(IX)

par un halogénure de benzyle,
à faire réagir le composé obtenu, de formule (X)

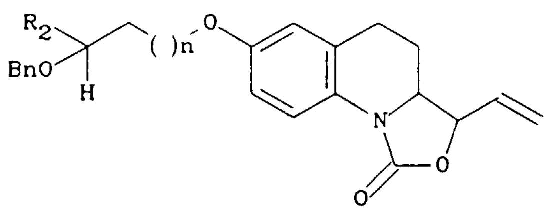

(X)

avec l'ozone puis un agent réducteur tel que le borohydrure de sodium pour obtenir le dérivé de formule (XI)

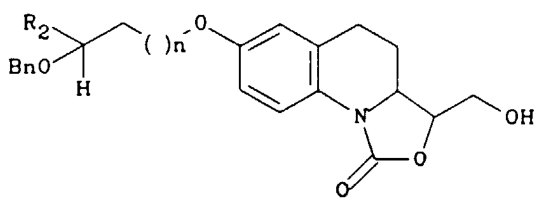

(XI)

qui est ensuite, soit déprotégé pour donner le composé de formule (I) correspondant, dans laquelle $R_1$ est un groupe hydroxyméthyle et $R_3$ un groupe hydroxyle,
soit traité par le sulfate de diméthyle pour donner le composé de formule (XII)

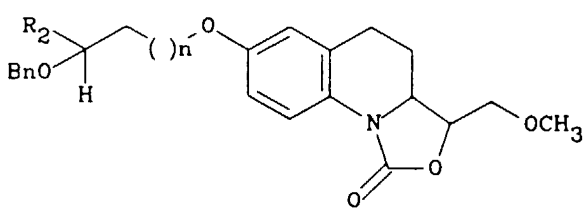

(XII)

qui est ensuite déprotégé pour donner le composé de formule (I) correspondant, dans laquelle $R_1$ est un groupe méthoxyméthyle et $R_3$ un groupe hydroxyle.

Les énantiomères et diastéréoisomères des composés de formule (I) sont préparés à partir des énantiomères ou diastéréoisomères des composés de formule (II) et/ou des énantiomères des composés de formule (III).
Les énantiomères et diastéréoisomères des composés de formule (II) sont eux-mêmes obtenus à partir des énantiomères et diastéréoisomères des composés de formule (IV) ou (V), préparés à partir du 6-méthoxy-2-méthoxycarbonyl-1,2,3,4-tétrahydro-quinoléine-1-carboxylate d'éthyle racémique, selon le procédé représenté en annexe 3.
Ce procédé comprend la séparation par hydrolyse enzymatique, des énantiomères du 6-méthoxy-2-méthoxycarbonyl-1,2,3,4-tétrahydro-quinoléine-1-carboxylate d'éthyle, consistant à traiter le composé racémique dans une solution tampon telle qu'un mélange de biphosphate de potassium et de phosphate de disodium ou dans un milieu biphasique tel que toluène/solution tampon, par un extrait enzymatique tel que l'estérase de foie de porc, les poudres acétoniques de foie

de cheval, de porc, de boeuf ou de lapin et, plus particulièrement, la poudre acétonique de foie de mouton (commercialisée par SIGMA), et à séparer par extraction, le S(-)-6-méthoxy-2-méthoxycarbonyl-1,2,3,4-tétrahydroquinoléine-1-carboxylate d'éthyle, du R(+)-2-carboxy-6-méthoxy-1,2,3,4-tétrahydro-quinoléine-1-carboxylate d'éthyle.

Le R(+)-2-carboxy-6-méthoxy-1,2,3,4-tétrahydro-quinoléine-1-carboxylate d'éthyle, traité par le chlorure de thionyle dans un solvant tel que le toluène, puis par le méthanol, donne le R(+)-6-méthoxy-2-méthoxycarbonyl-1,2,3,4-tétrahydroquinoléine-1-carboxylate d'éthyle.

Le R(+)- et le S(-)-6-méthoxy-2-méthoxycarbonyl-1,2,3,4-tétrahydro-quinoléine-1-carboxylate d'éthyle, traités par le borohydrure de lithium, conduisent respectivement aux énantiomères R(-) et S(+) de la 7-méthoxy-3,3*a*,4,5-tétrahydro-1*H*-oxazolo-[3,4-*a*]quinoléin-1-one (IV).

Le R(+)- et le S(-)-6-méthoxy-2-méthoxycarbonyl-1,2,3,4-tétrahydroquinoléine-1-carboxylate d'éthyle, traités par l'hydrure de diisobutylaluminium dans un solvant tel que le toluène, conduisent respectivement aux R(+)- et S(-)-2-formyl-6-méthoxy-1,2,3,4-tétrahydroquinoléine-1-carboxylate d'éthyle, qui, par réaction avec un organomagnésien de formule $R_1MgX$ dans laquelle $R_1$ représente un groupe éthényle, méthyle ou phényle et X un atome d'halogène, dans un solvant tel que tétrahydrofuranne, suivie d'une réaction avec le méthanolate de sodium dans un solvant tel que le toluène puis séparation chromatographique, donne d'une part, les diastéréoisomères (-), de configuration [3(*R*),3*a*(*R*)] et [3(*S*),3*a*(*R*)], et d'autre part les diastéréoisomères (+), de configuration [3(*S*),3*a*(*S*)] et [3(*R*),3*a*(*S*)], des composés de formule (V).

Le 6-méthoxy-2-méthoxycarbonyl-1,2,3,4-tétrahydroquinoléine-1-carboxylate d'éthyle racémique, utilisé comme produit de départ, peut être préparé par réaction de la 6-méthoxyquinoléine avec le cyanure de potassium ou le cyanure de triméthylsilyle et le chlorure de benzoyle, dans un solvant tel que le dichlorométhane, réaction de la 1-benzoyl-2-cyano-6-méthoxy-1,2-dihydroquinoléine obtenue, avec l'acide bromhydrique dans de l'acide acétique puis avec l'ammoniaque et enfin avec l'acide acétique, traitement de la 2-carboxy-6-méthoxyquinoléine obtenue, par le chlorure de thionyle dans un solvant tel que le toluène, puis par le méthanol, pour obtenir la 6-méthoxy-2-méthoxycarbonylquinoléine, qui est réduite par de l'hydrogène en présence d'oxyde de platine et d'éthanol chlorhydrique dans du méthanol, pour donner la 6-méthoxy-2-méthoxycarbonyl-1,2,3,4-tétrahydroquinoléine, qui est ensuite traitée par le chloroformiate d'éthyle dans un solvant tel que le dichlorométhane, en présence de carbonate de potassium.

Les exemples suivants illustrent la présente invention.

Exemple 1 : [3α,3*a*β,7(*R*)]-3-éthényl-7-[4,4,4-trifluoro-3-hydroxybutoxy]-3,3*a*,4,5-tétrahydro-1*H*-oxazolo[3,4-*a*] quinoléin-1-one

1.1 cis- et trans-(±)-3-Éthényl-7-méthoxy-3,3*a*,4,5-tétrahydro-1*H*-oxazolo[3,4-a]quinoléin-1-one.

A une solution de 116,3 g (0,442 mol) de 2-formyl-6-méthoxy-1,2,3,4-tétrahydroquinoléine-1-carboxylate d'éthyle dans 800 ml de tétrahydrofuranne, refroidie à - 30°C et sous agitation, on ajoute sous argon, en 30 mn, 486 ml (0,486 mol) de bromure de vinylmagnésium 1M. On laisse le milieu réactionnel sous agitation pendant 2 h, puis on ajoute une solution aqueuse saturée de chlorure d'ammonium glacée. On extrait deux fois à l'acétate d'éthyle, on lave à l'eau, on sèche sur sulfate de sodium et on évapore le solvant sous pression réduite. On redissout l'huile résiduelle dans 340 ml de toluène et on chauffe au reflux pour éliminer les traces d'eau. On ajoute alors, à 90°C, 1 ml d'une solution de méthylate de sodium à 10 % dans le méthanol, et on porte de nouveau au reflux en distillant l'éthanol formé. On évapore à sec, on reprend le résidu dans de l'acétate d'éthyle, on lave à l'eau, puis on sèche la phase organique sur sulfate de sodium et on évapore le solvant sous pression réduite. Par chromatographie du produit sur colonne de silice avec un mélange 4/1 d'heptane et d'acétate d'éthyle, on obtient 32,9 g de dérivé trans (Point de fusion : 100°C) et 13,4 g de dérivé cis (Point de fusion : 134°C).

1.2. trans-(±)-3-Éthényl-7-hydroxy-3,3a,4,5-tétrahydro-1*H*-oxazolo[3,4-*a*]quinoléin-1-one

A une solution de 24,6 g (0,1 mol) de trans-(±)-3-éthényl-7-méthoxy-3,3*a*,4,5-tétrahydro-1*H*-oxazolo[3,4-a] quinoléin-1-one dans 280 ml de dichlorométhane, on ajoute goutte à goutte, à 0°C, 19 ml (0,20 mol) de tribromure de bore, puis, au bout de 1 h, on verse une solution aqueuse saturée de bicarbonate de sodium jusqu'à neutralité. On filtre puis on extrait le filtrat avec du dichlorométhane contenant 10 % de méthanol. On lave la phase organique à l'eau, on la sèche sur sulfate de sodium puis on évapore le solvant sous pression réduite. On triture ensuite le résidu solide dans un mélange 1/1 de dichlorométhane et de méthanol. On filtre et on sèche pour obtenir finalement 21,4 g de produit. Point de fusion : 216°C.

A partir de la cis-(±)-3-éthényl-7-méthoxy-3,3*a*,4,5-tétra hydro-1*H*-oxazolo[3,4-*a*]quinoléin-1-one, on a obtenu la cis-(±)-3-éthényl-7-hydroxy-3,3*a*,4,5-tétrahydro-1*H*-oxazolo[3,4-*a*]quinoléin-1-one.
Point de fusion : 250°C.

1.3. [3α,3*a*β,7(*R*)]-3-Éthényl-7-[4,4,4-trifluoro-3-hydroxy butoxy]-3,3*a*,4,5-tétrahydro-1*H*-oxazolo[3,4-*a*]quinoléin-1-one

A une solution de 21,2 g (0,092 mol) de trans-(±)-3-éthényl-7-hydroxy-3,3*a*,4,5-tétrahydro-1*H*-oxazolo[3,4-*a*] quinoléin-1-one dans 150 ml d'acétonitrile, on ajoute sous argon, à température ambiante, 25,4 g (0,183 mol) de carbonate de potassium puis 34,9 g (0,138 mol) de 1-iodo-3(*R*)-hydroxy-4,4,4-trifluorobutane. Au bout de 4 h, on dilue le mélange avec du dichlorométhane et on le lave à l'eau. On sèche la phase organique sur sulfate de sodium puis on évapore le solvant sous pression réduite. L'huile résiduelle est purifiée par chromatographie sur colonne de silice avec du chloroforme contenant 0 à 3 % de méthanol. Après cristallisation dans un mélange acétone/éther diisopropylique, on obtient 30 g de produit.
Point de fusion : 135,8°C.

<u>Exemple 2</u> : <u>[3α,3*a*β,7(*R*)]-3-hydroxyméthyl-7-[4,4,4-trifluoro-3-benzyloxybutoxy]-3,3*a*,4,5-tétrahydro-1*H*-oxazolo [3,4-*a*]quinoléin-1-one</u>

2.1. [3α,3*a*β,7(*R*)]-3-Éthényl-7-[4,4,4-trifluoro-3-benzyloxy butoxy]-3,3*a*,4,5-tétrahydro-1*H*-oxazolo[3,4-*a*]quinoléin-1-one

A une solution de 30,0 g (840 mmol) de [3α,3*a*β,7(*R*)]-3-éthényl-7-[4,4,4-trifluoro-3-hydroxybutoxy]-3,3*a*, 4,5-tétrahydro-1*H*-oxazolo[3,4-*a*]quinoléin-1-one dans 300 ml de toluène, on ajoute une solution de 13,4 g d'hydroxyde de sodium dans 13,4 ml d'eau, 2,7 g (8,4 mmol) de bromure de tétrabutylammonium puis 43,1 g (0,252 mol) de bromure de benzyle. On agite le mélange pendant 2 h à température ambiante, puis on extrait à l'acétate d'éthyle, on lave à l'eau, on sèche sur sulfate de sodium et on évapore le solvant sous pression réduite. On purifie le résidu huileux par chromatographie sur colonne de silice avec un mélange 1/1 d'heptane et de chloroforme. On obtient 35 g de produit sous forme d'huile.

2.2. [3α,3*a*β,7(*R*)]-3-Hydroxyméthyl-7-[4,4,4-trifluoro-3-benzyloxybutoxy]-3,3*a*,4,5-tétrahydro-1*H*-oxazolo[3,4-*a*] quinoléin-1-one

On fait barboter de l'ozone pendant 3 h, à - 30°C, dans une solution de 31,5 g (70,4 mmol) de [3α,3*a*β,7(*R*)]-3-éthényl-7-[4,4,4-trifluoro-3-benzyloxybutoxy]-3,3*a*,4,5-tétrahydro-1*H*-oxazolo[3,4-*a*]quinoléin-1-one dans 515 ml de dichlorométhane et 780 ml de méthanol. On chasse ensuite l'ozone par passage d'un courant d'azote et on ajoute 26,8 g (0,704 mol) de boro hydrure de sodium en maintenant la température à - 30°C. Au bout de 5 mn, on ajoute 21,8 g (0,352 mol) de sulfure de diméthyle et on laisse le mélange revenir à température ambiante. On lave ensuite à l'eau, on sèche la phase organique sur sulfate de sodium et on concentre la solution sous pression réduite. Le produit obtenu est purifié par chromatographie sur colonne de silice avec du dichlorométhane contenant 0 à 5 % de méthanol. Après recristallisation dans l'éther diéthylique, on obtient 26,5 g de produit.
Point de fusion : 111,6°C.

<u>Exemple 3</u> : <u>[3α,3*a*β,7(*R*)]-3-méthoxyméthyl-7-[4,4,4-trifluoro -3 -benzyloxybutoxy ] -3,3*a*,4,5 -tétrahydro-1*H*-oxazolo [3,4-a]quinoléin-1-one</u>

A une solution de 7,8 g (17 mmol) de [3α,3*a*β,7(*R*)]-3-hydroxy méthyl-7-[4,4,4-trifluoro-3-benzyloxybutoxy]-3,3*a*,4,5-tétra hydro-1*H*-oxazolo[3,4-*a*]quinoléin-1-one dans 85 ml de toluène, on ajoute 0,54 g (1,7 mmol) de bromure de tétrabutylammonium puis 6,4 g (51 mmol) de sulfate de diméthyle. On verse ensuite goutte à goutte, en 30 mn, une solution de 2,7 g (67 mmol) d'hydroxyde de sodium dans 2,7 ml d'eau. On agite pendant 30 mn, puis on dilue le milieu réactionnel avec de l'acétate d'éthyle, on extrait la phase organique, on la lave à l'eau, on la sèche sur sulfate de sodium et on évapore le solvant sous pression réduite. Par chromatographie sur colonne de silice avec un mélange 1/1 d'heptane et d'acétate d'éthyle, on isole 6,2 g de produit sous forme d'huile.

<u>Exemple 4</u> : <u>[3α,3*a*β,7(R)]-3-méthoxyméthyl-7-[4,4,4-trifluoro-3-hydroxybutoxyl-3,3*a*,4,5-tétrahydro-1*H*-oxazolo [3,4-a]quinoléin-1-one</u>

On hydrogène pendant 16 h, 2,8 g (6 mmol) de [3α,3*a*β,7(*R*)]-3-méthoxyméthyl-7-[4,4,4-trifluoro-3-benzyloxybutoxy]-3,3*a*,4,5-tétrahydro-1*H*-oxazolo[3,4-*a*]quinoléin-1-one dans 30 ml d'éthanol, en présence de traces d'acide chlorhydrique et de 0,6 g de charbon palladié à 10 % contenant 50 % d'eau. On filtre le mélange sur silice et on évapore le solvant sous pression réduite. On purifie le produit obtenu par chromatographie sur colonne de silice avec un mélange 1/1 d'heptane et d'acétate d'éthyle. Après recristallisation dans l'éther diéthylique, on obtient 1,4 g de produit.
Point de fusion : 94,8°C.

Exemple 5 : [3α,3*a*β,7(*S*)]-7-(3-hydroxybutoxy)-3-hydroxyméthyl-3,3*a*,4,5-tétrahydro-1*H*-oxazolo[3,4-*a*] quinoléin-1-one

5.1. trans-7-Benzyloxy-3-éthényl-3,3a,4,5-tétrahydro-1*H*-oxazolo[3,4-a]quinoléin-1-one

A un mélange de 10 g (0,043 mol) de trans-3-éthényl-7-hydroxy-3,3*a*,4,5-tétrahydro-1*H*-oxazolo-[3,4-*a*]quinoléin-1-one (obtenue à l'étape 2 de l'exemple 1) et de 12 g (0,086 mol) de carbonate de potassium dans 250 ml d'acétonitrile et 50 ml de diméthylformamide, on ajoute 8,9 g (0,052 mol) de bromure de benzyle. On agite le mélange à 80°C pendant 1 h, puis on filtre à chaud, on lave à l'acétonitrile et on évapore le filtrat à sec sous pression réduite. On reprend le résidu par de l'acétate d'éthyle, on le lave plusieurs fois à l'eau, puis on sèche la phase organique sur sulfate de sodium et on la concentre sous pression réduite. Après recristallisation dans l'éther diisopropylique, on obtient 12,8 g de produit.
Point de fusion : 96°C.

5.2. trans-7-Benzyloxy-3-hydroxyméthyl-3,3*a*,4,5-tétrahydro-1*H*-oxazolo[3,4-*a*]quinoléin-1-one

On fait barboter de l'ozone pendant 3 h 30, dans une solution de 12,5 g (0,039 mol) de trans-7-benzyloxy-3-éthényl-3,3a,4, 5-tétrahydro-1*H*-oxazolo[3,4-*a*]quinoléin-1-one dans 450 ml de dichlorométhane et 350 ml de méthanol, refroidie à - 40°C. On chasse ensuite l'excès d'ozone par un courant d'azote, on ajoute 14,8 g (0,39 mol) de borohydrure de sodium par petites portions puis 12,6 g (0,195 mol) de diméthylsulfure et on laisse le mélange une nuit à température ambiante. On extrait deux fois au dichlorométhane, on lave la phase organique à l'eau puis avec une solution saturée de chlorure de sodium, on la sèche et on évapore le solvant sous pression réduite. Après purification par chromatographie sur colonne de silice avec un mélange 95/5 de dichlorométhane et de méthanol, on obtient 10,9 g de produit.
Point de fusion : 144°C.

5.3. trans-7-Hydroxy-3-hydroxyméthyl-3,3*a*,4,5-tétrahydro-1*H*-oxazolo[3,4-*a*]quinoléin-1-one

On hydrogène pendant 1 h, 3,0 g (9,2 mmol) de trans-7-benzyl oxy-3-hydroxyméthyl-3,3*a*,4,5-tétrahydro-1*H*-oxazolo[3,4-*a*] quinoléin-1-one en solution dans 20 ml d'éthanol et 40 ml de tétrahydrofuranne, en présence de 1 g de charbon palladié à 10 % contenant 50 % d'eau. On filtre ensuite le mélange sur silice et on évapore le solvant sous pression réduite. On obtient 1,1 g de produit.
Point de fusion : 250°C.

5.4. [3α,3*a*β,7(S)]-7-(3-Hydroxybutoxy)-3-hydroxyméthyl-3,3*a*, 4,5-tétrahydro-1*H*-oxazolo[3,4-*a*]quinoléin-1-one

A une solution de 500 mg (2,13 mmol) de trans-7-hydroxy-3-hydroxyméthyl-3,3*a*,4,5-tétrahydro-1*H*-oxazolo [3,4-*a*]quinoléin-1-one dans 3 ml d'acétonitrile et 2 ml de diméthylformamide, on ajoute 590 mg (4,26 mmol) de carbonate de potassium, puis une solution de 623 mg (2,55 mmol) de p-toluènesulfonate de 3(S)-hydroxybutane dans 5 ml d'acétonitrile. On agite le mélange à 80°C pendant 2 h, puis on ajoute 20 ml d'eau et on extrait 2 fois avec de l'acétate d'éthyle. On sèche la phase organique sur sulfate de sodium puis on la concentre sous pression réduite. Le solide obtenu est chromatographié sur colonne de silice avec un mélange 1/1 d'acétate d'éthyle et de cyclohexane puis trituré dans l'éther diisopropylique. On obtient 420 mg de produit.
Point de fusion : 102°C.

Exemple 6 : [3(*S*),3*a*(*S*),7(*R*)]-(+)-3-méthoxyméthyl-7-[4,4,4-trifluoro-3-hydroxybutoxy]-3,3*a*,4,5-tétrahydro-1*H*-oxazolo [3,4-*a*]quinoléin-1-one

6.1. 1-Benzoyl-2-cyano-6-méthoxy-1,2-dihydroquinoléine

On mélange une solution de 10 g (63 mmol) de 6-méthoxy quinoléine dans 80 ml de dichlorométhane avec une solution de 12,4 g (188 mmol) de cyanure de potassium puis on ajoute lentement 14,5 ml (125 mmol) de chlorure

de benzoyle. On agite le mélange pendant 18 h, puis on sépare la phase organique et on extrait la phase aqueuse avec du dichlorométhane. On lave les phases organiques avec une solution aqueuse d'acide chlorhydrique à 5 %, puis avec de l'eau, avec une solution aqueuse d'hydroxyde de sodium et de nouveau avec de l'eau, on les sèche sur sulfate de sodium et on évapore le solvant sous pression réduite. On cristallise l'huile obtenue dans de l'éthanol à 95 %. On obtient 13,4 g de produit. Point de fusion : 124°C.

6.2. 2-Carboxy-6-méthoxyquinoléine

On ajoute 270 ml d'acide bromhydrique à 48 % à une solution de 217 g (0,747 mol) de 1-benzoyl-2-cyano-6-méthoxy-1,2-di-hydroquinoléine dans 270 ml d'acide acétique et on chauffe le mélange au reflux pendant 30 mn. On filtre, rince à l'éther diéthylique, puis on met le solide obtenu en suspension dans 2 1 d'eau et on chauffe à 90°C. On ajoute ensuite de l'ammoniaque jusqu'à pH = 8-9 et on filtre à chaud. On acidifie le filtrat à 50°C, en ajoutant de l'acide acétique jusqu'à pH = 4-5, puis on refroidit. On filtre le produit cristallisé, on le rince à l'eau puis on le recristallise dans 250 ml d'acide acétique et on le rince à l'éther diéthylique. On obtient 129 g de produit. Point de fusion : 187°C.

6.3. 6-Méthoxy-2-méthoxycarbonylquinoléine

On verse goutte à goutte, 230 ml (3,17 mol) de chlorure de thionyle sur une suspension de 129 g (0,635 mol) de 2-carbo xy-6-méthoxyquinoléine dans 1200 ml de toluène et on chauffe le mélange pendant 3 h 30. Après concentration de la solution sous pression réduite, on dissout le solide obtenu dans 300 ml de méthanol. On agite pendant 30 mn, puis on évapore le solvant sous pression réduite, on reprend le produit obtenu avec de l'éther diéthylique et on l'isole par filtration. Le solide obtenu est repris avec de l'acétate d'éthyle et de l'ammoniaque diluée, puis la phase organique est séparée, traitée au noir animal, filtrée et concentrée sous pression réduite. On obtient ainsi 90 g de produit.
Point de fusion : 129°C.

6.4. (±)-6-Méthoxy-2-méthoxycarbonyl-1,2,3,4-tétrahydro quinoléine

A une solution de 50 g (0,23 mol) de 6-méthoxy-2-méthoxy carbonylquinoléine dans 1000 ml de méthanol, on ajoute une solution 6N d'éthanol chlorhydrique jusqu'à pH = 1, puis on hydrogène pendant 18 h, en présence de 2,6 g d'oxyde de platine hydraté. On élimine ensuite le catalyseur par filtration et on évapore le solvant sous pression réduite. Après trituration du produit dans un mélange d'éther diisopropylique et d'éther de pétrole, on obtient 56 g de produit sous forme de chlorhydrate.
Point de fusion : 129°C.
Après traitement avec de l'ammoniaque diluée, extraction à l'acétate d'éthyle, séchage sur sulfate de sodium et évaporation du solvant sous pression réduite, on récupère la base.
Point de fusion : < 50°C.

6.5. (±)-6-Méthoxy-2-méthoxycarbonyl-1,2,3,4-tétrahydro quinoléine-1-carboxylate d'éthyle

A une solution de 69,3 g (0,313 mol) de (±)-6-méthoxy-2-méthoxycarbonyl-1,2,3,4-tétrahydroquinoléine et de 39 ml (0,407 mol) de chloroformiate d'éthyle dans 1400 ml de dichlorométhane, on ajoute 85 g (0,63 mol) de carbonate de potassium et on chauffe au reflux pendant 18 h. On filtre le mélange, on concentre le filtrat sous pression réduite et on chromatographie le résidu sur colonne de silice avec un mélange 3/7 d'acétate d'éthyle et de cyclohexane. On obtient 80,4 g de produit sous forme d'huile.

6.6. *S*-(-)-6-Méthoxy-2-méthoxycarbonyl-1,2,3,4-tétrahydroquinoléine-1-carboxylate d'éthyle et *R*-(+)-2-carboxy-6-méthoxy-1,2,3,4-tétrahydroquinoléine-l-carboxylate d'éthyle

On met en suspension 3 g (10,2 mmol) de (±)-6-méthoxy-2-méthoxycarbonyl-1,2,3,4-tétrahydroquinoléine-1-carboxylate d'éthyle dans 80 ml de tampon phosphate 0,01 M (biphosphate de potassium + phosphate de disodium), à pH = 7. On ajuste ensuite le pH à 7,3 par addition d'une solution aqueuse 1M d'hydroxyde de sodium et on ajoute 7,5 g de poudre acétonique de foie de mouton. On agite le mélange pendant 14 h à température ambiante, tout en maintenant le pH constant par addition d'une solution aqueuse 1M d'hydroxyde de sodium, puis on filtre le mélange réactionnel sur célite et on rince la célite avec environ 400 ml d'éther diéthylique. Après extraction de la phase aqueuse par 3 fois 400 ml d'éther diéthylique, les phases organiques sont réunies, séchées sur sulfate de magnésium et filtrées puis le solvant est évaporé sous vide. On obtient 1,5 g d'un produit huileux correspondant au *S*-(-)-6-méthoxy-2-méthoxycarbonyl-1,2,3,4-tétrahydro-quinoléine-1-carboxylate d'éthyle.

ee : 99 % par HPLC chirale
$[\alpha]_D^{20}$ = - 54°7 (c = 0,9 ; dichlorométhane)
On acidifie la phase aqueuse à pH = 4,5 par addition d'acide chlorhydrique à 10 %, puis on extrait avec 3 fois 100 ml d'éther diéthylique. On réunit les phases éthérées, on les sèche sur sulfate de magnésium, les filtre et on évapore le solvant sous vide. On obtient 1,04 g de *R*-(+)-2-carboxy-6-méthoxy-1,2,3,4-tétrahydroquinoléine-1-carboxylate d'éthyle. ee : 88 % par HPLC chirale
$[\alpha]_D^{20}$ = + 66°5 (c = 0,99 ; dichlorométhane).

6.7. *S*-(-)-2-Formyl-6-méthoxy-1,2,3,4-tétrahydroquinoléine-1 carboxylate d'éthyle

A une solution de 34,6 g (0,118 mol) de *S*-(-)-6-méthoxy-2-méthoxycarbonyl-1,2,3,4-tétrahydro-quinoléine-1-carboxylate d'éthyle dans 700 ml de toluène, on ajoute goutte à goutte, à - 70°C, 235 ml (0,234 mol) d'une solution 1,5 M d'hydrure de diisobutylaluminium dans du toluène. On agite pendant 15 mn à - 70°C, puis on verse lentement 17 ml de méthanol et on agite en laissant revenir à température ambiante. On ajoute 1,5 l d'une solution 1,5 M d'acide chlorhydrique, puis on sépare la phase organique et on extrait la phase aqueuse avec de l'éther diéthylique. Les phases organiques sont lavées à l'eau plusieurs fois, puis le solvant est évaporé sous pression réduite. On obtient 23,8 g de produit.
$[\alpha]_D^{20}$ = - 43,1° (c = 1 ; dichlorométhane)

6.8. [3(*S*),3*a*(*S*)]-(+)- et [3(*R*),3*a*(*S*)]-(+)-3-Éthényl-7-méthoxy-3,3*a*,4,5-tétrahydro-1*H*-oxazolo[3,4-*a*]quinoléin-1-one

On refroidit à - 40°C, une solution de 23,8 g (90 mmol) de *S*-(-)-2-formyl-6-méthoxy-1,2,3,4-tétrahydroquinoléine-1-carboxylate d'éthyle dans 260 ml de tétrahydrofuranne et on ajoute sous argon et sous agitation magnétique, en 1 h 30, 99 ml (99 mmol) de bromure de vinylmagnésium 1M. On agite ensuite pendant 1 h, puis on ajoute une solution aqueuse saturée de chlorure d'ammonium glacée. On extrait 2 fois avec de l'éther diéthylique, on lave à l'eau, on sèche sur sulfate de sodium et on évapore le solvant sous pression réduite. On obtient une huile que l'on redissout dans 172 ml de toluène et on chauffe au reflux pour éliminer les traces d'eau. On ajoute à 90°C, 0,8 ml d'une solution de méthylate de sodium à 10 % dans le méthanol. On porte à nouveau au reflux en distillant l'éthanol formé puis on laisse revenir à température ambiante et on chromatographie la solution sur colonne de silice avec un mélange 4/1 de cyclohexane et d'acétate d'éthyle. On obtient 5,3 g de composé [3(*S*),3*a*(*S*)], Point de fusion : 80-83°C.
$[\alpha]_D^{20}$ = + 54,6° (c = 1 ; dichlorométhane)
et 3 g de composé [3(*R*),3*a*(*S*)],
Point de fusion : 137-138°C ;
$[\alpha]_D^{20}$ = + 41°8 (c = 1 ; dichlorométhane),

6.9. [3(*S*),3*a*(*S*)]-(+)-3-Éthényl-7-hydroxy-3,3*a*,4,5-tétrahydro-1*H*-oxazolo[3,4-*a*]quinoléin-1-one

A une solution de 5,3 g (22 mmol) de [3(*S*),3*a*(*S*)]-(+)-3-éthényl-7-méthoxy-3,3*a*,4,5-tétrahydro-1*H*-oxazolo [3,4-*a*]quinoléin-1-one dans 13 ml de dichlorométhane, on ajoute goutte à goutte, à 0°C, 43 ml (43 mmol) d'une solution 1M de tribromure de bore dans le dichlorométhane. On agite pendant 30 mn, puis on ajoute de l'ammoniaque diluée jusqu'à neutralité. On ajoute ensuite 5 à 10 ml de méthanol, on concentre le milieu réactionnel de moitié sous pression réduite et on filtre. On rince le précipité à l'eau puis à l'éther diéthylique et on le sèche. On obtient 4,7 g de produit.
Point de fusion : 215°C.
$[\alpha]_D^{20}$ = + 64,8° (c = 1 ; diméthylsulfoxyde)

6.10. [3(*S*),3*a*(*S*),7(*R*)]-(+)-3-Éthényl-7-[4,4,4-trifluoro-3-hydroxybutoxy]-3,3*a*,4,5-tétrahydro-1*H*-oxazolo[3,4-*a*] quinoléin-1-one

A une solution de 4,7 g (20 mmol) de [3(*S*),3*a*(*S*)]-(+)-3-éthé nyl-7-hydroxy-3,3*a*,4,5-tétrahydro-1*H*-oxazolo [3,4-*a*]quinoléin-1-one dans 55 ml d'acétonitrile, on ajoute 5,6 g (41 mmol) de carbonate de potassium puis 9,1 g (31 mmol) de p-toluènesulfonate de 3(R)-hydroxy-4,4,4-trifluorobutane. On chauffe au reflux pendant 16 h, puis on dilue le mélange avec du dichlorométhane et on le lave à l'eau. On sèche ensuite la phase organique sur sulfate de sodium puis on évapore le solvant sous pression réduite. L'huile obtenue est purifiée par chromatographie sur colonne de silice. Après cristallisation dans l'éther diisopropylique, on obtient 6,0 g de produit.
Point de fusion : 145-146°C.
$[\alpha]_D^{20}$ = + 78,4° (c = 1 ; méthanol)

6.11. [3(*S*),3*a*(*S*),7(*R*)]-(+)-3-Éthényl-7-[4,4,4-trifluoro-3-benzyloxybutoxy]-3,3*a*,4,5-tétrahydro-1H-oxazolo[3,4-*a*] quinoléin-1-one

A une solution de 6,0 g (17 mmol) de [3(*S*),3*a*(*S*),7(*R*)]-(+)-3-éthényl-7-[4,4,4-trifluoro-3-hydroxybutoxy]-3,3*a*,4,5-tétra hydro-1*H*-oxazolo[3,4-*a*]quinoléin-1-one dans 60 ml de toluène, on ajoute une solution de 2,7 g (67 mmol) d'hydroxyde de sodium dans 2,7 ml d'eau, 0,53 g (1,7 mmol) de bromure de tétrabutylammonium, puis 6,0 ml (50 mmol) de bromure de benzyle. On agite le mélange pendant 16 h à température ambiante, puis on extrait à l'acétate d'éthyle, on lave à l'eau, on sèche sur sulfate de sodium et on évapore le solvant sous pression réduite. L'huile obtenue est purifiée par chromatographie sur colonne de silice avec un mélange 8/2 de cyclohexane et d'acétate d'éthyle. On obtient 7,1 g de produit sous forme d'huile, qui cristallise lentement. Point de fusion : 84°C.
$[\alpha]_D^{20}$ = + 111° (c = 1 ; dichlorométhane)

6.12. [3(*S*),3*a*(*S*),7(*R*)]-(+)-3-Hydroxyméthyl-7-[4,4,4-tri fluoro-3-benzyloxybutoxy]-3,3*a*,4,5-tétrahydro-1*H*-oxazolo [3,4-*a*]quinoléin-1-one

On fait barboter de l'ozone, à - 40°C pendant 2 h, dans une solution de 7,0 g (16 mmol) de [3(*S*),3*a*(*S*),7(*R*)]-(+)-3-éthé nyl-7-[4,4,4-trifluoro-3-benzyloxybutoxy]-3,3*a*,4,5-tétra hydro-1*H*-oxazolo[3,4-*a*]quinoléin-1-one dans 170 ml de dichlorométhane et 240 ml de méthanol. On chasse ensuite l'ozone par un courant d'azote, puis on ajoute 5,9 g (160 mmol) de borohydrure de sodium, à la même température. Au bout de 5 mn, on ajoute 5,7 ml (78 mmol) de diméthylsulfure, on laisse revenir le mélange à température ambiante puis on le lave à l'eau, on sèche la phase organique sur sulfate de sodium et on la concentre sous pression réduite. On triture le produit obtenu avec de l'éther diéthylique puis on filtre. On obtient 4,7 g de produit.
Point de fusion : 118°C.
$[\alpha]_D^{20}$ = + 111,1° (c = 1 ; dichlorométhane)

6.13. [3(*S*),3*a*(*S*),7(*R*)]-(+)-3-Méthoxyméthyl-7-[4,4,4-tri fluoro-3-benzyloxybutoxy]-3,3*a*,4,5-tétrahydro-1*H*-oxazolo[3,4-*a*]quinoléin-1-one

A une solution de 4,6 g (10 mmol) de [3(*S*),3*a*(*S*),7(*R*)]-(+)-3-hydroxyméthyl-7-[4,4,4-trifluoro-3-benzyloxybutoxy]-3,3*a*,4,5-tétrahydro-1*H*-oxazolo[3,4-*a*]quinoléin-1-one dans 60 ml de toluène, on ajoute 0,32 g (1 mmol) de bromure de tétrabutyl ammonium puis 7,7 g (61 mmol) de sulfate de diméthyle et une solution de 3,2 g (82 mmol) d'hydroxyde de sodium dans 3,2 ml d'eau. On agite le mélange pendant 1 h, on le dilue dans de l'acétate d'éthyle puis on extrait la phase organique, on la lave à l'eau, on la sèche sur sulfate de sodium et on évapore le solvant sous pression réduite. Par chromatographie sur colonne de silice avec un mélange 7/3 de cyclohexane et d'acétate d'éthyle, on obtient 6,2 g de produit sous forme d'huile.
$[\alpha]_D^{20}$ = + 115,4° (c = 1 ; dichlorométhane).

6.14. [3(*S*),3*a*(*S*),7(*R*)]-(+)-3-Méthoxyméthyl-7-[4,4,4-tri fluoro-3-hydroxybutoxy]-3,3*a*,4,5-tétrahydro-1*H*-oxazolo [3,4-*a*]quinoléin-1-one

On hydrogène pendant 1 h, une solution de 4,1 g (8,8 mmol) de [3(*S*),3*a*(*S*),7(*R*)]-(+)-3-méthoxyméthyl-7-[4,4,4-trifluoro-3-benzyloxybutoxy]-3,3*a*,4,5-tétrahydro-1*H*-oxazolo[3,4-*a*] quinoléin-1-one dans 80 ml d'éthanol, en présence de 0,8 g de charbon palladié à 10 % contenant 50 % d'eau et de traces d'éthanol chlorhydrique. On filtre ensuite le mélange sur silice et on évapore le solvant sous pression réduite. On cristallise le produit dans un mélange d'acétone et d'éther diisopropylique puis dans un mélange 97/3 d'éther diisopropylique et d'isopropanol. On obtient 1,0 g de produit. Point de fusion : 120,7-120,9°C.
$[\alpha]_D^{20}$ = + 105,4° (c = 1 ; méthanol)

<u>Exemple 7</u> : <u>[3(*R*),3*a*(*S*),7(*R*)]-(+)-3-méthoxyméthyl-7-[4,4,4-trifluoro-3-hydroxybutoxy]-3,3*a*,4,5-tétrahydro-1*H*-oxazolo [3,4-*a*]quinoléin-1-one</u>

7.1. [3(*R*),3*a*(*S*)]-(+)-3-éthényl-7-hydroxy-3,3*a*,4,5-tétrahydro-1*H*-oxazolo[3,4-*a*]quinoléin-1-one

On traite 2,0 g (8,0 mmol) de [3(*R*),3*a*(*S*)]-(+)-3-éthényl-7-méthoxy-3,3*a*,4,5-tétrahydro-1*H*-oxazolo[3,4-*a*]quinoléin-1-one (obtenue à l'étape 8 de l'exemple 6) dans les conditions décrites à l'étape 9 de l'exemple 6. On obtient 1,9 g de produit.
$[\alpha]_D^{20}$ = + 47,2° (c = 1, diméthylsulfoxyde).

7.2. [3(*R*),3*a*(*S*),7(*R*)]-(+)-3-Éthényl-7-[4,4,4-trifluoro-3-hydroxybutoxy]-3,3*a*,4,5-tétrahydro-1*H*-oxazolo[3,4-*a*] quinoléin-1-one

On traite une solution de 1,9 g (8,0 mmol) de [3(*R*),3*a*(*S*)]-(+) -3-éthényl-7-hydroxy-3,3*a*,4,5-tétrahydro-1*H*-oxazolo[3,4-*a*]quinoléin-1-one dans 26 ml d'acétonitrile et 10 ml de diméthylformamide, dans les conditions décrites à l'étape 10 de l'exemple 6.
On obtient 2,6 g de produit.
Point de fusion : 143-145°C.
$[\alpha]_D^{20}$ = + 60,2° (c = 1 ; méthanol)

7.3. [3(*R*),3*a*(*S*),7(*R*)]-(+)-3-Éthényl-7-[4,4,4-trifluoro-3-benzyloxybutoxy]-3,3*a*,4,5-tétrahydro-1*H*-oxazolo[3,4-*a*] quinoléin-1-one

On traite 2,5 g (7,0 mmole) de [3(*R*),3*a*(*S*),7(*R*)]-(+)-3-éthényl-7-[4,4,4-trifluoro-3-hydroxybutoxy]-3,3*a*,4,5-tétrahydro-1H-oxazolo[3,4-*a*]quinoléin-1-one dans les conditions décrites à l'étape 11 de l'exemple 6.
On obtient 3,0 g de produit.
Point de fusion : 73-74°C.
$[\alpha]_D^{20}$ = + 102,7° (c = 1 ; dichlorométhane)

7.4. [3(*R*),3*a*(*S*),7(*R*)]-(+)-3-Hydroxyméthyl-7-[4,4,4-trifluoro-3-benzyloxybutoxy]-3,3*a*,4,5-tétrahydro-1*H*-oxa zolo [3,4-*a*]quinoléin-1-one

On traite 3,0 g (6,7 mmol) de [3(*R*),3*a*(*S*),7(*R*)]-(+)-3-éthényl-7-[4,4,4-trifluoro-3-benzyloxybutoxy]-3,3*a*,4,5-tétrahydro-1H-oxazolo[3,4-*a*]quinoléin-1-one, dans les conditions décrites à l'étape 12 de l'exemple 6.
On obtient 2,1 g de produit.
Point de fusion : 107°C.
$[\alpha]_D^{20}$ = + 63,8° (c = 1 ; dichlorométhane).

7.5. [3(*R*),3*a*(*S*),7(*R*)]-(+)-3-Méthoxyméthyl-7-[4,4,4-trifluoro-3-benzyloxybutoxy]-3,3*a*,4,5-tétrahydro-1*H*-oxazolo [3,4-*a*]quinoléin-1-one

On traite une solution de 2,1 g (4,7 mmol) de [3(*R*),3*a*(*S*), 7(*R*)]-(+)-3-hydroxyméthyl-7-[4,4,4-trifluoro-3-benzyloxy butoxy]-3,3*a*,4,5-tétrahydro-1*H*-oxazolo[3,4-*a*]quinoléin-1-one dans 30 ml de toluène et 3 ml de dichlorométhane, dans les conditions décrites à l'étape 13 de l'exemple 6. On obtient 1,8 g de produit sous forme d'huile.
$[\alpha]_D^{20}$ = + 64,8° (c = 1 ; dichlorométhane).

7.6. [3(*R*),3*a*(*S*),7(*R*)]-(+)-3-Méthoxyméthyl-7-[4,4,4-trifluoro-3-hydroxybutoxy]-3,3*a*,4,5-tétrahydro-1*H*-oxa zolo [3,4-*a*]quinoléin-1-one

On traite 1,8 g (3,9 mmol) de [3(*R*),3*a*(*S*),7(*R*)]-(+)-3-méthoxyméthyl-7-[4,4,4-trifluoro-3-benzyloxybutoxy]-3,3*a*,4,5-tétrahydro-1*H*-oxazolo[3,4-*a*]quinoléin-1-one dans les conditions décrites à l'étape 14 de l'exemple 6.
On obtient 0,9 g de produit.
Point de fusion : 165,3-165,5°C.
$[\alpha]_D^{20}$ = + 16,7° (c = 1 ; méthanol)

<u>Exemple 8</u> : <u>[3(*R*),3*a*(*R*)7(*R*))-(-)-3-méthoxyméthyl-7-[4,4,4-trifluoro-3-hydroxybutoxy]-3,3*a*,4,5-tétrahydro-1*H*-oxazolo [3,4-*a*]quinoléin-1-one</u>

8.1. R-(+)-6-Méthoxy-2-méthoxycarbonyl-1,2,3,4-tétrahydro quinoléine-1-carboxylate d'éthyle

On verse, goutte à goutte, à - 40°C, 21,8 ml (302 mmol) de chlorure de thionyle dans 170 ml de méthanol. Au bout de 10 mn, on ajoute 16,9 g (60,4 mmol) de *R*-(+)-2-carboxy-6-méthoxy-1,2,3,4-tétrahydroquinoléine-l-carboxylate d'éthyle (obtenu à l'étape 6 de l'exemple 6), on agite le mélange pendant 3 h en laissant remonter la température de - 40 à 0°C, puis on le verse dans un mélange de glace et d'eau et on ajoute de l'ammoniaque jusqu'à neutralité. On extrait avec de l'acétate d'éthyle, on lave la phase organique à l'eau, on la sèche sur sulfate de sodium et on évapore le solvant. On obtient 18 g de produit sous forme d'huile.
$[\alpha]_D^{20}$ = + 52° (c = 1 ; dichlorométhane)

8.2. *R*-(+)-2-Formyl-6-méthoxy-1,2,3,4-tétrahydroquinoléine-1-carboxylate d'éthyle

On traite 18 g (0,061 mol) de *R*-(+)-6-méthoxy-2-méthoxy carbonyl-1,2,3,4-tétrahydroquinoléine-1-carboxylate d'éthyle dans les conditions décrites à l'étape 7 de l'exemple 6. On obtient 11,6 g de produit.
$[\alpha]_D^{20}$ = + 67,9° (c = 1 ; dichlorométhane).

8.3. [3(*R*),3*a*(*R*)]-(-)- et [3(*S*),3*a*(*R*)]-(-)-3-Éthényl-7-méthoxy-3,3*a*,4,5-tétrahydro-1*H*-oxazolo[3,4-*a*]quinoléin-1-one

On traite 11,5 g (43,6 mmole) de *R*-(+)-2-formyl-6-méthoxy-1,2, 3,4-tétrahydroquinoléine-1-carboxylate d'éthyle dans les conditions décrites à l'étape 8 de l'exemple 6.
On obtient 4,0 g de composé [3(*R*),3*a*(*R*)],
Point de fusion : 80°C.
$[\alpha]_D^{20}$ = - 48,8° (c = 1 ; dichlorométhane)
et 2,2 g de composé [3(*S*),3*a*(*R*)],
Point de fusion : 140°C,
$[\alpha]_D^{20}$ = - 39° (c = 1, dichlorométhane),

8.4. [3(*R*),3*a*(*R*)]-(-)-3-Éthényl-7-hydroxy-3,3*a*,4,5-tétrahydro-1*H*-oxazolo[3,4-*a*]quinoléin-1-one

On traite 4,0 g (16 mmol) de [3(*R*),3*a*(*R*)]-(-)-3-éthényl-7-méthoxy-3,3*a*,4,5-tétrahydro-1*H*-oxazolo[3,4-*a*]quinoléin-1-one dans les conditions décrites à l'étape 9 de l'exemple 6. On obtient 2,5 g de produit.
$[\alpha]_D^{20}$ = - 45,2° (c = 1 ; diméthylsulfoxyde).

8.5. [3(*R*),3*a*(*R*),7(*R*)]-(-)-3-Éthényl-7-[4,4,4-trifluoro-3-hydroxybutoxy]-3,3*a*,4,5-tétrahydro-1*H*-oxazolo[3,4-*a*] quinoléin-1-one

On traite une solution de 2,35 g (10 mmol) de [3(*R*),3*a*(*R*)]-(-)-3-éthényl-7-hydroxy-3,3*a*,4,5-tétrahydro-1*H*-oxazolo[3,4-*a*]quinoléin-1-one dans 25 ml d'acétonitrile et 10 ml de diméthylformamide, dans les conditions décrites à l'étape 10 de l'exemple 6.
On obtient 2,5 g de produit.
Point de fusion : 92°C.
$[\alpha]_D^{20}$ = - 31,4° (c = 1 ; dichlorométhane).

8.6. [3(*R*),3*a*(*R*),7(*R*)]-(+)-3-Éthényl-7-[4,4,4-trifluoro-3-benzyloxybutoxy]-3,3*a*,4,5-tétrahydro-1*H*-oxazolo[3,4-*a*] quinoléin-1-one

On traite de 1,82 g (5,09 mmol) de [3(*R*),3*a*(*R*),7(*R*)]-(-)-3-éthényl-7-[4,4,4-trifluoro-3-hydroxybutoxy]-3,3*a*, 4,5-tétrahydro-1*H*-oxazolo[3,4-*a*]quinoléin-1-one dans les conditions décrites à l'étape 11 de l'exemple 6.
On obtient 2,1 g de produit sous forme d'huile.
$[\alpha]_D^{20}$ = + 28,9° (c = 1 ; dichlorométhane).

8.7. [3(*R*),3*a*(*R*),7(*R*)]-(+)-3-Hydroxyméthyl-7-[4,4,4-trifluoro-3-benzyloxybutoxy]-3,3*a*,4,5-tétrahydro-1H-oxa zolo [3,4-a]quinoléin-1-one

On traite 2,1 g (4,7 mmol) de [3(*R*),3*a*(*R*),7(*R*)]-(+)-3-éthényl-7-[4,4,4-trifluoro-3-benzyloxybutoxy]-3,3*a*, 4,5-tétrahydro-1*H*-oxazolo[3,4-*a*]quinoléin-1-one dans les conditions décrites à l'étape 12 de l'exemple 6. On obtient 1,4 g de produit.
Point de fusion : 110°C.
$[\alpha]_D^{20}$ = + 15,5° (c = 1 ; dichlorométhane)

8.8. [3(*R*),3*a*(*R*),7(*R*)]-(+)-3-Méthoxyméthyl-7-[4,4,4-trifluoro-3-benzyloxybutoxy]-3,3*a*,4,5-tétrahydro-1*H*-oxazolo [3,4-*a*]quinoléin-1-one

On traite 1,4 g (3,1 mmol) de [3(*R*),3*a*(*R*),7(*R*)]-(+)-3-hydroxyméthyl-7-[4,4,4-trifluoro-3-benzyloxybutoxy]-3,3*a*,4,5-tétrahydro-1*H*-oxazolo[3,4-*a*]quinoléin-1-one dans les conditions décrites à l'étape 13 de l'exemple 6. On obtient 1,0 g de produit sous forme d'huile.
$[\alpha]_D^{20}$ = + 15,8° (c = 1 ; dichlorométhane)

8.9. [3(*R*),3*a*(*R*),7(*R*)]-(-)-3-Méthoxyméthyl-7-[4,4,4-trifluoro-3-hydroxybutoxy]-3,3*a*,4,5-tétrahydro-1*H*-oxazolo[3,4-*a*] quinoléin-1-one

On traite 1,0 g (2,2 mmol) de [3(*R*),3*a*(*R*),7(*R*)]-(+)-3-métho xyméthyl-7-[4,4,4-trifluoro-3-benzyloxybutoxy]-3,3*a*,4,5-tétrahydro-1*H*-oxazolo[3,4-*a*]quinoléin-1-one dans les conditions décrites à l'étape 14 de l'exemple 6. Après évaporation du solvant sous pression réduite et chromatographie sur colonne de silice avec un mélange 1/1 de cyclohexane et d'acétate d'éthyle, on obtient 0,65 g de produit. Point de fusion : 90°C.
$[\alpha]_D^{20}$ = - 35,6° (c = 1 ; méthanol)

<u>Exemple 9</u> : <u>[3(*S*),3*a*(*R*),7(*R*)]-(+)-3-méthoxyméthyl-7-[4,4,4-trifluoro-3-hydroxybutoxy]-3,3*a*,4,5-tétrahydro-1H-oxazolo [3,4-*a*]quinoléin-1 -one</u>

9.1. [3(*S*),3*a*(*R*)]-(-)-3-Éthényl-7-hydroxy-3,3*a*,4,5-tétrahydro-1*H*-oxazolo[3,4-*a*]quinoléin-1-one

On traite 2,2 g (9,0 mmol) de [3(*S*),3*a*(*R*)]-(-)-3-éthényl-7-méthoxy-3,3*a*,4,5-tétrahydro-1*H*-oxazolo[3,4-*a*]quinoléin-1-one (obtenue à l'étape 3 de l'exemple 8), dans les conditions décrites à l'étape 9 de l'exemple 6. On obtient 1,8 g de produit.
$[\alpha]_D^{20}$ = - 50,9° (c = 1 ; diméthylsulfoxyde)

9.2. [3(*S*),3*a*(*R*),7(*R*)]-(-)-3-Éthényl-7-[4,4,4-trifluoro-3-hydroxybutoxy]-3,3*a*,4,5-tétrahydro-1*H*-oxazolo[3,4-*a*]quinoléin-1-one

On traite 1,7 g (7,5 mmol) de [3(*S*),3*a*(*R*)]-(-)-3-éthényl-7-hydroxy-3,3*a*,4,5-tétrahydro-1*H*-oxazolo[3,4-*a*]quinoléin-1-one, dans les conditions décrites à l'étape 10 de l'exemple 6. Après cristallisation dans l'éther diéthylique, on obtient 1,8 g de produit.
Point de fusion : 145°C.
$[\alpha]_D^{20}$ = - 16,4° (c = 1, méthanol)

9.3. [3(*S*),3*a*(*R*),7(*R*)]-(+)-3-Éthényl-7-[4,4,4-trifluoro-3-benzyloxybutoxy]-3,3*a*,4,5-tétrahydro-1*H*-oxazolo[3,4-*a*]quinoléin-1-one

On traite 1,77 g (4,95 mmol) de [3(*S*),3*a*(*R*),7(*R*)]-(-)-3-éthényl-7-[4,4,4-trifluoro-3-hydroxybutoxy]-3,3*a*,4,5-tétra hydro-1*H*-oxazolo[3,4-*a*]quinoléin-1-one, dans les conditions décrites à l'étape 11 de l'exemple 6. Après purification par chromatographie sur colonne de silice avec un mélange 1/9 de cyclohexane et de chloroforme, on obtient 2,0 g de produit. $[\alpha]_D^{20}$ = + 42,7° (c = 1 : dichlorométhane)

9.4. [3(*S*),3*a*(*R*),7(*R*)]-(+)-3-Hydroxyméthyl-7-[4,4,4-trifluoro-3-benzyloxybutoxy]-3,3*a*,4,5-tétrahydro-1*H*-oxazolo [3,4-*a*]quinoléin-1-one

On traite 1,9 g (4,2 mmol) de [3(*S*),3*a*(*R*),7(*R*)]-(+)-3-éthényl-7-[4,4,4-trifluoro-3-benzyloxybutoxy]-3,3*a*,4,5-tétrahydro-1*H*-oxazolo[3,4-*a*]quinoléin-1-one, dans les conditions décrites à l'étape 12 de l'exemple 6. Après concentration de la phase organique sous pression réduite, on obtient 1,7 g de produit.
Point de fusion : 98°C.
$[\alpha]_D^{20}$ = + 69,5° (c = 1 ; dichlorométhane)

9.5. [3(*S*),3*a*(*R*),7(*R*)]-(+)-3-Méthoxyméthyl-7-[4,4,4-trifluoro-3-benzyloxybutoxy]-3,3*a*,4,5-tétrahydro-1*H*-oxa zolo [3,4-*a*]quinoléin-1-one

On traite 1,68 g (3,72 mmol) de [3(*S*),3*a*(*R*),7(*R*)]-(+)-3-hydroxyméthyl-7-[4,4,4-trifluoro-3-benzyloxybutoxy]-3,3*a*,4,5-tétrahydro-1*H*-oxazolo[3,4-*a*]quinoléin-1-one, dans les conditions décrites à l'étape 13 de l'exemple 6. Par chromatographie sur colonne de silice avec un mélange 6/4 de cyclohexane et d'acétate d'éthyle, on obtient 1,35 g de produit sous forme d'huile.
$[\alpha]_D^{20}$ = + 61,6° (c = 1 ; dichlorométhane)

9.6. [3(*S*),3*a*(*R*),7(*R*)]-(+)-3-Méthoxyméthyl-7-[4,4,4-trifluoro-3-hydroxybutoxy]-3,3*a*,4,5-tétrahydro-1*H*-oxa zolo [3,4-*a*]quinoléin-1-one

On traite 1,29 g (2,77 mmol) de 3(*S*),3*a*(*R*),7(*R*)]-(+)-3-méthoxyméthyl-7-[4,4,4-trifluoro-3-benzyloxybutoxy]-3,3*a*,4,5-tétrahydro-1*H*-oxazolo[3,4-*a*]quinoléin-1-one, dans les conditions décrites à l'étape 14 de l'exemple 6. On obtient 0,33 g de composé.
Point de fusion : 103,6-103,8°C.
$[\alpha]_D^{20}$ = + 49,1° (c = 1 ; méthanol)

Exemple 10 : 7(*R*)-(4,4,4-trifluoro-3-hydroxybutoxy)-3,3*a*,4,5-tétrahydro-1*H*-oxazolo[3,4-*a*]quinoléin-1-one

10.1. 7-Méthoxy-3,3*a*,4,5-tétrahydro-1*H*-oxazolo[3,4-*a*] quinoléin-1-one

A une solution de 10,9 g (41,4 mmol) de 2-formyl-6-méthoxy-1,2,3,4-tétrahydroquinoléine-1-carboxylate d'éthyle dans 100 ml de méthanol refroidie à 0°C, on ajoute 2,2 g (41,4 mmol) de borohydrure de potassium par portions. On agite pendant 1 h puis on hydrolyse le milieu, on le dilue à l'eau et on extrait à l'éther diéthylique. On lave ensuite la phase organique à l'eau, on la sèche sur sulfate de sodium et on évapore le solvant sous pression réduite. On redissout l'huile obtenue dans 90 ml de toluène, on chauffe la solution à reflux pour éliminer les traces d'eau puis on ajoute à 90°C, une quantité catalytique de méthylate de sodium à 10 % dans du méthanol. On chauffe de nouveau à reflux pour éliminer l'éthanol formé puis on évapore le solvant. On reprend le résidu par de l'acétate d'éthyle et on le lave à l'eau, puis on sèche la phase organique sur sulfate de sodium et on la concentre sous pression réduite. Après chromatographie sur colonne de silice avec un mélange 4/1 d'heptane et d'acétate d'éthyle, on obtient 5,0 g de produit.
Point de fusion : 99°C.

10.2. 7-Hydroxy-3,3*a*,4,5-tétrahydro-1*H*-oxazolo[3,4-*a*]quinoléin-1-one

A une solution de 4,6 g (21 mmol) de 7-méthoxy-3,3*a*,4,5-tétrahydro-1*H*-oxazolo[3,4-*a*]quinoléin-1-one dans 50 ml de dichlorométhane, on ajoute goutte à goutte, à 0°C, 4,0 ml (42 mmol) de tribromure de bore. Au bout d'une heure, on hydrolyse le milieu par addition d'ammoniaque jusqu'à neutralité. On filtre ensuite le précipité formé et on le sèche sous vide. On obtient 3,1 g de produit.
Point de fusion : > 260°C.

10.3. 7(*R*)-(4,4,4-Trifluoro-3-hydroxybutoxy)-3,3*a*,4,5-tétrahydro-1*H*-oxazolo[3,4-*a*]quinoléin-1-one

A une solution de 2,0 g (9,7 mmol) de 7-hydroxy-3,3*a*,4,5-tétrahydro-1*H*-oxazolo[3,4-*a*]quinoléin-1-one dans 10 ml d'acétonitrile et 10 ml de diméthylformamide, on ajoute 4,3 g (15 mmol) de tosylate de 3(R)-hydroxy-4,4,4-trifluorobutyle et 4,0 g (29 mmol) de carbonate de potassium. On agite pendant 4 h à 90°C, puis on dilue le mélange avec de l'acétate d'éthyle et on le lave à l'eau. On sèche la phase organique sur sulfate de sodium puis on évapore le solvant sous pression réduite. Après purification de l'huile obtenue par chromatographie sur colonne de silice avec un mélange 4/1 d'heptane et d'acétate d'éthyle, on obtient 1,9 g de produit. Point de fusion : 188°C.

Exemple 11 : *S*(+)-7-(4,4,4-trifluorobutoxy)-3,3*a*,4,5-tétrahydro-1*H*-oxazolo[3,4-*a*]quinoléin-1-one

11.1. *S*(+)-7-Méthoxy-3,3*a*,4,5-tétrahydro-1*H*-oxazolo[3,4-*a*]quinoléin-1-one

A une solution de 8,0 g (27 mmol) de *S*(-)-6-méthoxy-2-méthoxycarbonyl-1,2,3,4-tétrahydroquinoléine-1-carboxylate d'éthyle (obtenu à l'étape 6 de l'exemple 6) dans 80 ml de diglyme, on ajoute 0,90 g (41 mmol) de borohydrure de lithium par portions. On agite le mélange pendant 3 heures à 50°C puis on le verse dans de l'eau et on extrait le produit avec de l'acétate d'éthyle. On sèche la phase organique sur sulfate de sodium, on la concentre sous pression réduite et on triture le résidu dans de l'éther de pétrole contenant un peu d'alcool isopropylique. On obtient 4,4 g de produit. Point de fusion : 112°C.
$[\alpha]_D^{20}$ = + 63,7° (c = 1 ; dichlorométhane)
Selon le même procédé, à partir du *R*(+)-6-méthoxy-2-méthoxycarbonyl-1,2,3,4-tétrahydroquinoméine-1-carboxylate d'éthyle, on a obtenu la *R*(-)-7-méthoxy-3,3*a*,4,5-tétrahydro-1*H*-oxazolo[3,4-*a*]quinoléin-1-one.
Point de fusion : 110°C.
$[\alpha]_D^{20}$ = - 40,1° (c = 1 ; dichlorométhane).

11.2. *S*(+)-7-Hydroxy-3,3*a*,4,5-tétrahydro-1*H*-oxazolo[3,4-*a*]quinoléin-1-one

A une solution de 4,3 g (20 mmol) de *S*(+)-7-méthoxy-3,3*a*,4,5-tétrahydro-1*H*-oxazolo[3,4-*a*]quinoléin-1-one dans 40 ml de dichlorométhane refroidie à 0°C, on ajoute goutte à goutte, à 0°C, 39 ml (39 mmol) d'une solution 1M de tribromure de bore dans le dichlorométhane. On agite pendant 1 heure en laissant remonter la température, puis

on ajoute une solution aqueuse d'ammoniaque jusqu'à neutralité, on filtre le précipité formé et on le sèche. On obtient 3,0 g de produit.
Point de fusion : > 250°C.
$[\alpha]_D^{20}$ = + 51,4° (c = 1 ; diméthylsulfoxyde).

Selon le même procédé, à partir de la *R*(-)-7-méthoxy-3,3*a*,4,5-tétrahydro-1*H*-oxazolo[3,4-*a*]quinoléin-1-one, on a obtenu la *R*(-)-7-hydroxy-3,3*a*,4,5-tétrahydro-1*H*-oxazolo[3,4-*a*]quinoléin-1-one.
Point de fusion : > 250°C.
$[\alpha]_D^{20}$ = - 46° (c = 1 ; diméthylsulfoxyde).

11.3. *S*(+)-7-(4,4,4-Trifluorobutoxy)-3,3*a*,4,5-tétrahydro-1*H*-oxazolo[3,4-*a*]quinoléin-1-one

A une solution de 1,9 g (9,3 mmol) de *S*(+)-7-hydroxy-3,3*a*,4, 5-tétrahydro-1*H*-oxazolo[3,4-*a*]quinoléin-1-one dans un mélange de 10 ml de diméthylformamide et 40 ml d'acétonitrile, on ajoute 2,7 g (13 mmol) de 1-bromo-4,4,4-trifluorobutane et 2,6 g (18 mmol) de carbonate de potassium. On chauffe pendant 3 heures à 90°C puis on dilue le mélange avec de l'acétate d'éthyle et on le lave à l'eau. On sèche ensuite la phase organique sur sulfate de sodium, on la concentre sous pression réduite et on chromatographie le résidu sur colonne de silice avec du dichlorométhane contenant 0,5 % de méthanol. Après recristallisation dans l'alcool isopropylique, on obtient 2,1 g de produit.
Point de fusion : 121,3-121,4°C.
$[\alpha]_D^{20}$ = + 33,8° (c= 1 ; dichlorométhane).

Exemple 12 : cis-(±)-3-phényl-7-(4,4,4-trifluorobutoxy)-3,3*a*,4,5-tétrahydro-1*H*-oxazolo[3,4-*a*]quinoléin-1-one

12.1. cis- et trans-(±)-7-Méthoxy-3-phényl-3,3*a*,4,5-tétrahydro-1*H*-oxazolo[3,4-*a*]quinoléin-1-one

A partir d'une solution de 2-formyl-6-méthoxy-1,2,3,4-tétrahydroquinoléine-1-carboxylate d'éthyle dans le méthanol, refroidie à 0°C, et de bromure de phénylmagnésium, traités dans des conditions analogues à celles de l'étape 1 de l'exemple 1, on obtient le dérivé cis,
Point de fusion : 99°C, et
le dérivé trans,
Point de fusion : 126°C.

12.2. cis-(+)-7-Hydroxy-3-phényl-3,3*a*,4,5-tétrahydro-1*H* oxazolo[3,4-*a*]quinoléin-1-one

A partir de la cis-(±)-7-méthoxy-3-phényl-3,3*a*,4,5-tétra hydro-1*H*-oxazolo[3,4-*a*]quinoléin-1-one, traitée dans les conditions de l'étape 2 de l'exemple 1, on obtient la cis(±)-7-hydroxy-3-phényl-3,3*a*,4,5-tétrahydro-1*H*-oxazolo [3,4-*a*] quinoléin-1-one.
Point de fusion : 242°C.

A partir de la trans-(±)-7-méthoxy-3-phényl-3,3*a*,4,5-tétra hydro-1*H*-oxazolo[3,4-*a*]quinoléin-1-one, on a obtenu la trans(±)-7-hydroxy-3-phényl-3,3*a*,4,5-tétrahydro-1*H*-oxazolo[3,4-*a*] quinoléin-1-one.
Point de fusion : 216°C.

12.3. cis-(±)-3-Phényl-7-(4,4,4-trifluorobutoxy)-3,3*a*,4,5-tétrahydro-1*H*-oxazolo[3,4-*a*]quinoléin-1-one

A une solution de 0,65 g (2,3 mmol) de cis-(±)-7-hydroxy-3-phényl-3,3*a*,4,5-tétrahydro-1*H*-oxazolo[3,4-*a*]quinoléin-1-one dans 15 ml de diméthylformamide, on ajoute 0,66 g (3,5 mmol) de 1-bromo-4,4,4-trifluorobutane et 0,64 g (4,6 mmol) de carbonate de potassium. On chauffe pendant 4 heures à 90°C, puis on dilue le mélange avec de l'acétate d'éthyle et on le lave à l'eau. On sèche ensuite la phase organique sur sulfate de sodium, on la concentre sous pression réduite et on chromatographie le résidu sur colonne de silice avec du cyclohexane contenant 20 % d'acétate d'éthyle. Après trituration dans l'éther diisopropylique, on obtient 0,60 g de produit.
Point de fusion : 129,5°C.

Exemple 13 : cis-(±)-3-méthyl-7-(4,4,4-trifluorobutoxy)-3,3*a*,4,5-tétrahydro-1*H*-oxazolo[3,4-*a*]quinoléin-1-one

13.1. cis-(±)-7-Méthoxy-3-méthyl-3,3*a*,4,5-tétrahydro-1*H*-oxazolo[3,4-*a*]quinoléin-1-one

A partir d'une solution de 2-formyl-6-méthoxy-1,2,3,4-tétrahydroquinoléine-1-carboxylate d'éthyle dans l'éther diéthylique, refroidie à 0°C, et de bromure de méthyl-magnésium, traités dans des conditions analogues à celles de l'étape 1 de l'exemple 1, on obtient le dérivé cis, Point de fusion : 138-139°C, et le dérivé trans,

Point de fusion : 122-123°C.

13.2. cis-(±)-7-Hydroxy-3-méthyl-3,3*a*,4,5-tétrahydro-1*H*-oxazolo[3,4-*a*]quinoléin-1-one

A partir de la cis-(±)-7-méthoxy-3-méthyl-3,3*a*,4,5-tétra hydro-1*H*-oxazolo[3,4-*a*]quinoléin-1-one, traitée dans les conditions de l'étape 2 de l'exemple 1, on obtient la cis-(±)-7-hydroxy-3-méthyl-3,3*a*,4,5-tétrahydro-1*H*-oxazolo [3,4-*a*]quinoléin-1-one.
Point de fusion : 258-259°C.
A partir de la trans-(±)-7-méthoxy-3-méthyl-3,3*a*,4,5-tétra hydro-1*H*-oxazolo[3,4-*a*]quinoléin-1-one, on a obtenu la trans-(±)-7-hydroxy-3-méthyl-3,3*a*,4,5-tétrahydro-1*H*-oxazolo [3,4-*a*] quinoléin-1-one.
Point de fusion : 240-241°C.

13.3. cis-(±)-3-Méthyl-7-(4,4,4-trifluorobutoxy)-3,3*a*,4,5-tétrahydro-1*H*-oxazolo[3,4-*a*]quinoléin-1-one

A une solution de 0,70 g (3,2 mmol) de cis-(±)-7-hydroxy-3-méthyl-3,3*a*,4,5-tétrahydro-1*H*-oxazolo[3,4-*a*]quinoléin-1-one dans 15 ml de diméthylformamide, on ajoute 0,90 g (4,8 mmol) de 1-bromo-4,4,4-trifluorobutane et 0,90 g (6,4 mmol) de carbonate de potassium. On chauffe à 90°C pendant 4 heures, puis on dilue le mélange avec de l'acétate d'éthyle et on le lave à l'eau. On sèche ensuite la phase organique sur sulfate de sodium, on la concentre sous pression réduite et on chromatographie le résidu sur colonne de silice avec du cyclohexane contenant 20 % d'acétate d'éthyle. Après trituration dans l'éther diisopropylique, on obtient 0,80 g de produit.
Point de fusion : 79,1-79,2°C.

<u>Exemple 14</u> : <u>[3α,3aβ,7(R)]-3-éthyl-7-(4,4,4-trifluoro-3-hydroxybutoxy)-3,3*a*,4,5-tétrahydro-1*H*-oxazolo[3,4-*a*]quinoléin-1-one</u>

On hydrogène pendant 3 h, 0,86 g (2,4 mmol) de [3α,3*a*β,7(*R*)]-3-éthényl-7-[4,4,4-trifluoro-3-hydroxybutoxy]-3,3*a*,4,5-tétrahydro-1*H*-oxazolo[3,4-*a*]quinoléin-1-one (obtenue à l'exemple 1) dans 20 ml de méthanol, en présence de 0,2 g de charbon palladié à 5 % contenant 50 % d'eau. On filtre ensuite le mélange et on le concentre à sec sous pression réduite. Après purification de l'huile obtenue par chromatographie sur colonne de silice avec un mélange 95/5 de dichlorométhane et de méthanol et cristallisation dans l'éther diisopropylique, on obtient 0,47 g de produit. Point de fusion : 120-131°C.

<u>Exemple 15</u> : <u>[3(*S*),3*a*(*S*),7(*S*)]-(+)-3-méthoxyméthyl-7-[4,4,4-trifluoro-3-hydroxybutoxy]-3,3*a*,4,5-tétrahydro-1*H*-oxazolo[3,4-*a*]quinoléin-1-one</u>

15.1. [3(*S*),3*a*(*S*)]-(+)-7-Benzyloxy-3-éthényl-3,3*a*,4,5-tétrahydro-1*H*-oxazolo[3,4-*a*]quinoléin-1-one

A partir de la [3(*S*),3*a*(*S*)]-(+)-3-éthényl-7-hydroxy-3,3*a*,4,5-tétrahydro-1*H*-oxazolo[3,4-*a*]quinoléin-1-one (obtenue à l'étape 9 de l'exemple 6), traitée dans les conditions décrites à l'étape 1 de l'exemple 5, on obtient la [3(*S*), 3*a* (*S*)-(±)-7-benzyloxy-3-éthényl-3,3*a*,4,5-tétrahydro-1*H*-oxazolo[3,4-*a*]quinoléin-1-one.
Point de fusion : 86-90°C.
$[\alpha]_D^{20}$ = + 71,1° (c = 1 ; dichlorométhane).
Selon le même procédé, les composés suivants ont été obtenus :

- la [3(*S*),3*a*(*R*)]-(-)-7-benzyloxy-3-éthényl-3,3*a*,4,5-tétrahydro-1*H*-oxazolo[3,4-*a*]quinoléin-1-one
  Point de fusion : 102°C
  $[\alpha]_D^{20}$ = - 45,2°.

- la [3(*R*),3*a*(*S*)]-(+)-7-benzyloxy-3-éthényl-3,3*a*,4,5-tétrahydro-1*H*-oxazolo[3,4-*a*]quinoléin-1-one
  Point de fusion : 98°C
  $[\alpha]_D^{20}$ = + 45,7°.

- la [3(*R*),3*a*(*R*)]-(-)-7-benzyloxy-3-éthényl-3,3*a*,4,5-tétrahydro-1*H*-oxazolo[3,4-*a*]quinoléin-1-one
  Point de fusion : 84°C
  $[\alpha]_D^{20}$ = - 62,1°.

15.2. [3(*S*),3*a*(*S*)]-(+)-7-Benzyloxy-3-hydroxyméthyl-3,3*a*,4,5-tétrahydro-1*H*-oxazolo[3,4-*a*]quinoléin-1-one

A partir du composé obtenu à l'étape précédente, traité dans les conditions décrites à l'étape 2 de l'exemple 5, on obtient le [3(*S*),3*a*(*S*)]-(+)-7-benzyloxy-3-hydroxyméthyl-3,3*a*,4,5-tétrahydro-1*H*-oxazolo[3,4-*a*]quinoléin-1-one. Point de fusion : 120-140°C
$[\alpha]_D^{20}$ = + 73,8° (c = 1 ; diméthylsulfoxyde).

Selon le même procédé, les composés suivants ont été obtenus :

- la [3(*S*),3*a*(*R*)]-(-)-7-benzyloxy-3-hydroxyméthyl-3,3*a*,4,5-tétrahydro-1*H*-oxazolo[3,4-*a*]quinoléin-1-one, sous forme de gomme.
  $[\alpha]_D^{20}$ = - 4,2°.

- la [3(*R*),3*a*(*S*)]-(+)-7-benzyloxy-3-hydroxyméthyl-3,3*a*,4,5-tétrahydro-1*H*-oxazolo[3,4-*a*]quinoléin-1-one
  Point de fusion : 138-140°C
  $[\alpha]_D^{20}$ = + 4,8°.

- la [3(*R*),3*a*(*R*)]-(-)-7-benzyloxy-3-hydroxyméthyl-3,3*a*,4,5-tétrahydro-1*H*-oxazolo[3,4-*a*]quinoléin-1-one, sous forme de gomme.
  $[\alpha]_D^{20}$ = - 54,6°.

15.3. [3(*S*),3*a*(*S*)]-(+)-7-Benzyloxy-3-méthoxyméthyl-3,3*a*,4,5-tétrahydro-1*H*-oxazolo[3,4-*a*]quinoléin-1-one

A une solution de 1,7 g (0,052 mol) de [3(*S*),3*a*(*S*)]-(+)-7-benzyloxy-3-hydroxyméthyl-3,3*a*,4,5-tétrahydro-1*H*-oxazolo[3,4-*a*]quinoléin-1-one dans 100 ml de toluène et 100 ml de dichlorométhane, on ajoute 0,17 g (0,005 mol) de bromure de tétraméthylammonium puis 3,0 ml de sulfate de diméthyle, puis une solution de 1,7 g (0,041 mol) d'hydroxyde de sodium dans 1,7 ml d'eau. On agite le mélange pendant 1 h 30 mn, puis on le dilue avec de l'acétate d'éthyle. On sépare ensuite la phase organique, on la lave à l'eau, on la sèche sur sulfate de sodium et on évapore le solvant sous pression réduite. Après chromatographie sur colonne de silice avec un mélange 3/2 de cyclohexane et d'acétate d'éthyle, on obtient 1,2 g de produit.
Point de fusion : 118-120°C
$[\alpha]_D^{20}$ = + 76,7° (c = 1 ; dichlorométhane).

Selon le même procédé, les composés suivants ont été obtenus :

- la [3(*S*),3*a*(*R*)]-(-)-7-benzyloxy-3-méthoxyméthyl-3,3*a*,4,5-tétrahydro-1*H*-oxazolo[3,4-*a*]quinoléin-1-one
  Point de fusion : 99°C
  $[\alpha]_D^{20}$ = - 2,2°.

- la [3(*R*),3*a*(*S*)]-(+)-7-benzyloxy-3-méthoxyméthyl-3,3*a*,4,5-tétrahydro-1*H*-oxazolo[3,4-*a*]quinoléin-1-one
  Point de fusion : 96-98°C
  $[\alpha]_D^{20}$ = + 1,1°.

- la [3(*R*),3*a*(*R*)]-(-)-7-benzyloxy-3-méthoxyméthyl-3,3*a*,4,5-tétrahydro-1*H*-oxazolo[3,4-*a*]quinoléin-1-one
  Point de fusion : 122°C
  $[\alpha]_D^{20}$ = - 74°.

15.4. [3(*S*),3*a*(*S*)]-(+)-7-Hydroxy-3-méthoxyméthyl-3,3*a*,4,5-tétrahydro-1*H*-oxazolo[3,4-*a*]quinoléin-1-one

On hydrogène pendant 5 h, sous pression et à température ambiante, une solution de 1,2 g (0,0035 mol) de [3(*S*),3*a*(*S*)]-(+)-7-benzyloxy-3-méthoxyméthyl-3,3*a*,4,5-tétrahydro-1*H*-oxazolo[3,4-*a*]quinoléin-1-one dans 50 ml de méthanol et 40 ml de tétrahydrofuranne, en présence de 0,25 g de palladium sur charbon à 10 % contenant 50 % d'eau. On filtre ensuite le mélange sur silice et on évapore le solvant sous pression réduite. On obtient 0,9 g de produit.
Point de fusion : 172-176°C.
$[\alpha]_D^{20}$ = + 99° (c = 1 ; diméthylsulfoxyde).

Selon le même procédé, les composés suivants ont été obtenus :

- la [3(*S*),3*a*(*R*)]-(+)-7-hydroxy-3-méthoxyméthyl-3,3*a*,4,5-tétrahydro-1*H*-oxazolo[3,4-*a*]quinoléin-1-one
  Point de fusion : 170°C
  $[\alpha]_D^{20}$ = + 11,1°.

- la [3(*R*),3*a*(*S*)]-(-)-7-hydroxy-3-méthoxyméthyl-3,3*a*,4,5-tétrahydro-1*H*-oxazolo[3,4-*a*]quinoléin-1-one
  Point de fusion : 166°C
  $[\alpha]_D^{20}$ = - 8,2°.

- la [3(*R*),3*a*(*R*)]-(-)-7-hydroxy-3-méthoxyméthyl-3,3*a*,4,5-tétrahydro-1*H*-oxazolo[3,4-*a*]quinoléin-1-one
  Point de fusion : 180°C
  $[\alpha]_D^{20}$ = - 93,3°.

15.5. [3(*S*),3*a*(*S*),7(*S*)]-(+)-3-Méthoxyméthyl-7-[4,4,4-trifluoro-3-hydroxybutoxy]-3,3*a*,4,5-tétrahydro-1*H*-oxazolo [3,4-*a*]quinoléin-1-one

A partir de 0,86 g (3,45 mmol) de [3(*S*),3*a*(*S*)]-(+)-7-hydroxy-3-méthoxyméthyl-3,3*a*,4,5-tétrahydro-1*H*-oxazolo[3,4-*a*] quinoléin-1-one et 1,54 g (5,18 mmol) de p-toluènesulfonate de 4,4,4-trifluoro-3(*S*)-hydroxybutane, traités dans les conditions de l'étape 4 de l'exemple 5, on obtient 0,38 g de produit sous forme d'huile.
$[\alpha]_D^{20}$ = + 33,2° (c = 1 ; méthanol).

Les composés selon l'invention sont regroupés dans le tableau suivant avec leurs caractéristiques physiques.

## Tableau

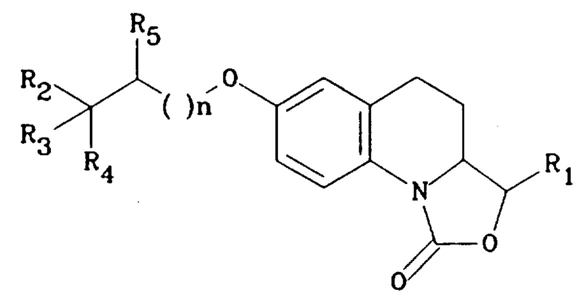

| N° | $R_1$ | $R_2(R_3)C(R_4)-CH(R_5)-(CH_2)_n-$ | Config. | F (°C) | $[\alpha]_D^{20}$ |
|---|---|---|---|---|---|
| 1 | | | 3α,3aα,7(*R*) | 131,7 | |
| 2 | | | 3α,3aß,7(*R*) | 135,8 | |
| 3 | -CH=CH2 | 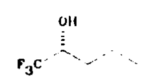 | 3(*S*),3a(*S*),7(*R*) | 145-146 | + 78,4° |
| 4 | | | 3(*R*),3a(*S*),7(*R*) | 143-145 | + 60,2° |
| 5 | | | 3(*R*),3a(*R*),7(*R*) | 92 | - 31,4° |
| 6 | | | 3(*S*),3a(*R*),7(*R*) | 145 | - 16,4° |

| N° | $R_1$ | $R_2(R_3)C(R_4)-CH(R_5)-(CH_2)_n-$ | Config. | F (°C) | $[\alpha]_D^{20}$ |
|---|---|---|---|---|---|
| 7 | | | 3α,3aα,7(*R*) | 101,7 | |
| 8 | | | 3α,3aß,7(*R*) | 111,6 | |
| 9 | -CH2OH | 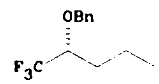 | 3(*S*),3a(*S*),7(*R*) | 118 | + 111,1° |
| 10 | | | 3(*R*),3a(*S*),7(*R*) | 107 | + 63,8° |
| 11 | | | 3(*R*),3a(*R*),7(*R*) | 110 | + 15,5° |
| 12 | | | 3(*S*),3a(*R*),7(*R*) | 98 | + 69,5° |
| 13 | -CH2OH | 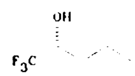 | 3α,3aα,7(*R*) | 122,5 | |
| 14 | | | 3α,3aß,7(*R*) | 147,6 | |
| 15 | | | 3α,3aα,7(*R*) | huile | |
| 16 | | | 3α,3aß,7(*R*) | huile | |
| 17 | -CH2OCH3 | 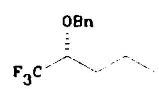 | 3(*S*),3a(*S*),7(*R*) | huile | + 115,4° |
| 18 | | | 3(*R*),3a(*S*),7(*R*) | huile | + 64,8° |
| 19 | | | 3(*R*),3a(*R*),7(*R*) | huile | + 15,8° |
| 20 | | | 3(*S*),3a(*R*),7(*R*) | huile | + 61,6° |

| N° | $R_1$ | $R_2(R_3)C(R_4)-CH(R_5)-(CH_2)_n-$ | Config. | F (°C) | $[\alpha]_D^{20}$ |
|---|---|---|---|---|---|
| 21 | | | 3α,3aα,7(*R*) | 163,4 | |
| 22 | | | 3α,3aß,7(*R*) | 94,8 | |
| 23 | -CH2OCH3 | 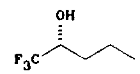 | 3(*S*),3a(*S*),7(*R*) | 120,7-120,9 | + 105,4° |
| 24 | | | 3(*R*),3a(*S*),7(*R*) | 165,3-165,5 | + 16,7° |
| 25 | | | 3(*R*),3a(*R*),7(*R*) | 90 | - 35,6° |
| 26 | | | 3(*S*),3a(*R*),7(*R*) | 103,6-103,8 | + 49,1° |
| 27 | | | 3α,3aß,7(*S*) | 95,5 | |
| 28 | | | 3(*R*),3a(*R*),7(*S*) | 118,8 | - 107,0° |
| 29 | -CH2OCH3 | 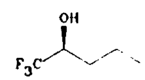 | 3(*R*),3a(*S*),7(*S*) | 103,8-103,9 | - 50,4° |
| 30 | | | 3(*S*),3a(*R*),7(*S*) | 163,5-163,7 | - 17,5° |
| 31 | | | 3(*S*),3a(*S*),7(*S*) | huile | + 33,2° |
| 32 | -CH=CH2 | 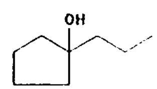 | (cis) (±) | 115 | |
| 33 | | | (trans) (±) | 110 | |
| 34 | -CH2OH | 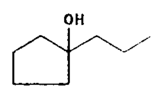 | (cis) (±) | huile | |
| 35 | | | (trans) (±) | huile | |

| N° | $R_1$ | $R_2(R_3)C(R_4)-CH(R_5)-(CH_2)_n-$ | Config. | F (°C) | $[\alpha]_D^{20}$ |
|---|---|---|---|---|---|
| 36 | -CH2OCH3 | 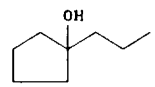 | (cis) (±) | 120,6 | |
| 37 | | | (trans) (±) | 129 | |
| 38 | -CH2OCH3 | 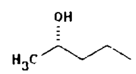 | 3α,3aα,7(*R*) | 70 | |
| 39 | | | 3α,3aß,7(*R*) | 104 | |
| 40 | -CH2OH | 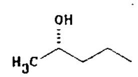 | 3α,3aß,7(*R*) | 102 | |
| 41 | -CH=CH2 | 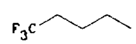 | (cis) (±) | 74,4-74,8 | |
| 42 | | | (trans) (±) | 98,2 | |
| 43 | | | 3(*S*),3a(*S*) | 68-72 | + 41,1° |
| 44 | | | 3(*S*),3a(*R*) | 105 | - 34,1° |
| 45 | | | 3(*R*),3a(*S*) | 98-102 | + 31,2° |
| 46 | | | 3(*R*),3a(*R*) | 80 | - 45,1° |
| 47 | -CH2OH | 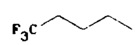 | (3α,3aα)(±) | 138,8 | |
| 48 | | | 3(*S*),3a(*S*) | 128 | + 56,2° |
| 49 | | | 3(*S*),3a(*R*) | 124 | + 12,1° |
| 50 | | | 3(*R*),3a(*S*) | 124 | - 12,2° |
| 51 | | | 3(*R*),3a(*R*) | 130 | - 57,4° |

| N° | $R_1$ | $R_2(R_3)C(R_4)-CH(R_5)-(CH_2)_n-$ | Config. | F (°C) | $[\alpha]_D^{20}$ |
|---|---|---|---|---|---|
| 52 | -CH2OCH3 | 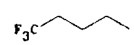 | (3α,3aα)(±) | 91 | |
| 53 | | | (3α,3aß)(±) | 81 | |
| 54 | | | 3(*S*),3a(*S*) | 83,0-83,3 | + 72,9° |
| 55 | | | 3(*R*),3a(*S*) | 110,6 | - 16,9° |
| 56 | | | 3(*R*),3a(*R*) | 81,6-82,2 | - 69,2° |
| 57 | | | 3(*S*),3a(*R*) | 111,0-111,2 | + 17,2° |
| 58 | -CH2OH | 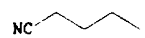 | (3α,3aß)(±) | 142 | |
| 59 | -CH2OCH3 | 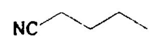 | (3α,3aα)(±) | 95 | |
| 60 | | | (3α,3aß)(±) | 63,5 | |
| 61 | -CH2OH | 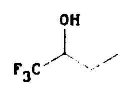 | (3α,3aß)(±) | 132 | |
| 62 | -CH2OCH3 | 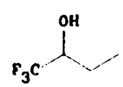 | (3α,3aß)(±) | huile | |
| 63 | H | 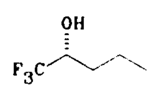 | 3a(*R*,*S*),7(*R*) | 188 | |

| N° | $R_1$ | $R_2(R_3)C(R_4)-CH(R_5)-(CH_2)_n-$ | Config. | F (°C) | $[\alpha]_D^{20}$ |
|---|---|---|---|---|---|
| 64<br>65<br>66 | H | 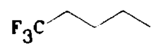 | 3a(*R*,*S*)<br>3a(*S*)<br>3a(*R*) | 119,2<br>121,3-121,4<br>121,3 | <br>+ 33,8°<br>- 31,3° |
| 67 | H | 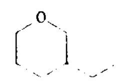 | 3a(*R*,*S*) | 115 | |
| 68 | H | 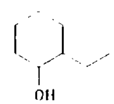 | 3a(*R*,*S*) | 139 | |
| 69 | H | 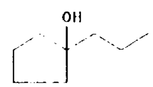 | 3a(*R*,*S*) | 120,9 | |
| 70 | H | 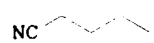 | 3a(*R*,*S*) | 120,9 | |
| 71 | C2H5 | 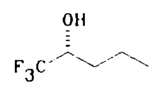 | 3α,3aß,7(*R*) | 120-131 | |

| N° | $R_1$ | $R_2(R_3)C(R_4)-CH(R_5)-(CH_2)_n-$ | Config. | F (°C) | $[\alpha]_D^{20}$ |
|---|---|---|---|---|---|
| 72 | C2H5 | 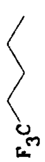 | trans (±) | 78-82 | |
| 73 | CH3 | 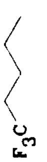 | trans (±) | 79,1-79,2 | |
| 74 | | | cis (±) | 63,9-64,0 | |
| 75 | 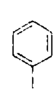 | 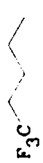 | trans (±) | 129,5 | |
| 76 | | | cis (±) | 86 | |

Les composés de l'invention ont fait l'objet d'essais pharmacologiques permettant de déterminer leur pouvoir inhibiteur de la monoamine oxydase A et de la monoamine oxydase B.

Les mesures des activités MAO-A et MAO-B in vitro ont été réalisées en utilisant comme source d'enzyme un homogénat de cerveau de rat, selon la méthode décrite par C. Fowler et M. Strolin-Benedetti, dans J. Neurochem., 40, 1534-1541 (1983).

Le dosage standard consiste à homogénéiser le cerveau de rat dans 20 volumes de tampon phosphate 0,1 M (pH = 7,4) et à préincuber 100 µl d'homogénat (5 mg de tissu) à 37°C pendant 20 minutes, en l'absence ou en présence de différentes concentrations en inhibiteur étudié. La réaction est démarrée par l'addition de [$^{14}$C]sérotonine ([$^{14}$C]5HT, concentration finale 125 µM) pour la mesure de l'activité de la MAO-A ou de [$^{14}$C]phényléthylamine ([$^{14}$C] PEA, concentration finale 8 µM) pour la mesure de l'activité MAO-B, dans un volume final de 500 µl. Après 5 minutes d'incubation pour la [$^{14}$C]5HT et 1 minute d'incubation pour la [$^{14}$C]PEA, la réaction est arrétée par addition de 200 µl d'acide chlorhydrique 4N. Les métabolites radioactifs issus de la désamination oxydative sont alors séparés du substrat non transformé, par extraction en phase organique, et quantifiés par comptage de la radio-activité.

Les activités inhibitrices vis à vis de la MAO-A et de la MAO-B sont données respectivement par les constantes d'inhibition Ki (MAO-A) et Ki (MAO-B).

Pour les composés de l'invention, les Ki (MAO-A) varient entre 0,4 et 28 nM et les Ki (MAO-B) entre 0,7 et 1000 nM.

Certains composés de l'invention sont des inhibiteurs sélectifs de la MAO-A, le rapport Ki(MAO-B)/Ki(MAO-A) pouvant être compris entre 10 et 1000.

D'autres sont cependant des inhibiteurs mixtes de la MAO-A et de la MAO-B, le rapport Ki(MAO-B)/Ki(MAO-A) pouvant être compris entre 0,1 et 10.

L'activité inhibitrice de la MAO a également été mise en évidence in vivo par le test de la potentialisation du L-5-hydroxytryptophane (L-5HTP) chez le rat, selon le protocole décrit par M. Jalfre et col. dans Arch. Int. Pharmacodyn. 259, 194-221 (1982).

Ce test est réalisé dans les conditions suivantes : des lots de rats (10 pour chaque dose), traités par voie orale, par différentes doses de produit à tester ou de véhicule, reçoivent par voie intrapéritonéale, 60 minutes après, le L-5HTP, à la dose de 100 mg/kg, dose qui n'induit pas par elle-même le syndrome sérotoninergique (tremblements généralisés) chez les animaux témoins.

Les tremblements généralisés sont cotés en tout ou rien 30 minutes après l'administration du L-5HTP. Pour chaque dose de produit testé, les résultats sont exprimés en pourcentage d'animaux présentant des tremblement généralisés. La dose qui induit des tremblements chez 50 % des animaux ($DE_{50}$), affectée de son intervalle de confiance à 95 %, est ensuite calculée à partir de la droite de régression qui relie l'effet (pourcentage obtenu pour chaque dose) au logarithme de la dose.

Pour les composés de l'invention, la $DE_{50}$ varie entre 0,2 et 1,1 mg/kg, confirmant ainsi l'activité inhibitrice de la MAO trouvée in vitro.

Par ailleurs, l'évaluation de la toxicité in vitro des composés de l'invention sur hépatocytes de rat et de primate non humain après administration unique démontre une très bonne tolérance dans la limite des concentrations testées (jusqu'à 100 µM).

Les résultats obtenus montrent que les composés de l'invention peuvent être utilisés pour la préparation de médicaments inhibiteurs sélectifs de la MAO-A ou inhibiteurs mixtes de la MAO-A et de la MAO-B, ces médicaments trouvant leur emploi en thérapeutique notamment dans le traitement des états dépressifs, des attaques de panique, des phobies, de l'anxiété, de la migraine, des déficits cognitifs liés à l'âge ou aux démences et dans la prévention et le traitement des maladies neurodégénératives comme la maladie de Parkinson et et la maladie d'Alzheimer, et des accidents cérébro-vasculaires.

Les composés de l'invention peuvent être présentés, en association avec des excipients, sous forme de compositions formulées en vue de l'administration par voie orale, parentérale ou rectale, par exemple sous forme de comprimés, dragées, capsules, solutions, suspensions ou suppositoires.

Par voie orale, la dose de principe actif administrée par jour peut varier entre 1 et 100 mg/kg, en une ou plusieurs prises. Par voies parentérale et rectale, elle peut varier entre 1 et 100 mg/kg.

## Annexe 1

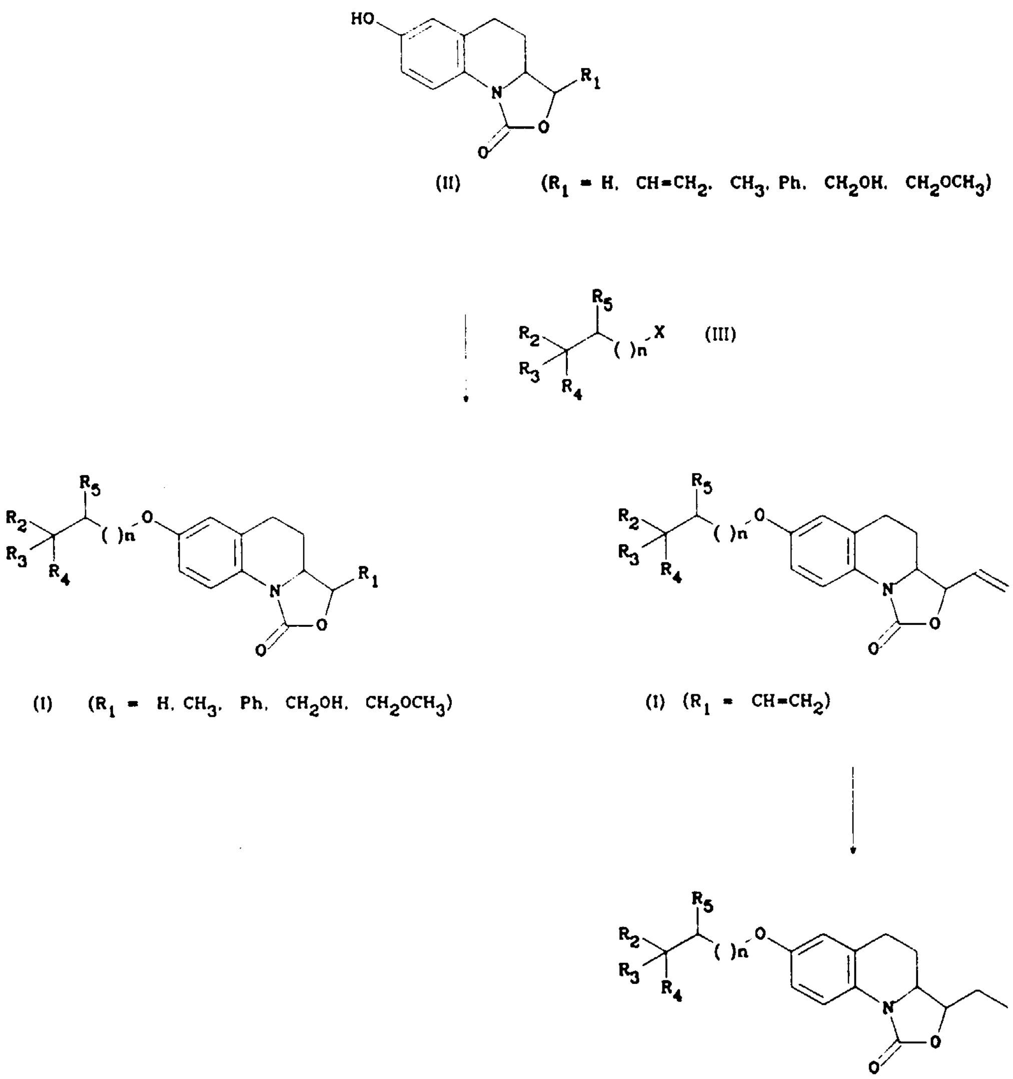

(II) ($R_1$ = H, $CH=CH_2$, $CH_3$, Ph, $CH_2OH$, $CH_2OCH_3$)

(III)

(I) ($R_1$ = H, $CH_3$, Ph, $CH_2OH$, $CH_2OCH_3$)

(I) ($R_1$ = $CH=CH_2$)

(I) ($R_1$ = $C_2H_5$)

## Annexe 2

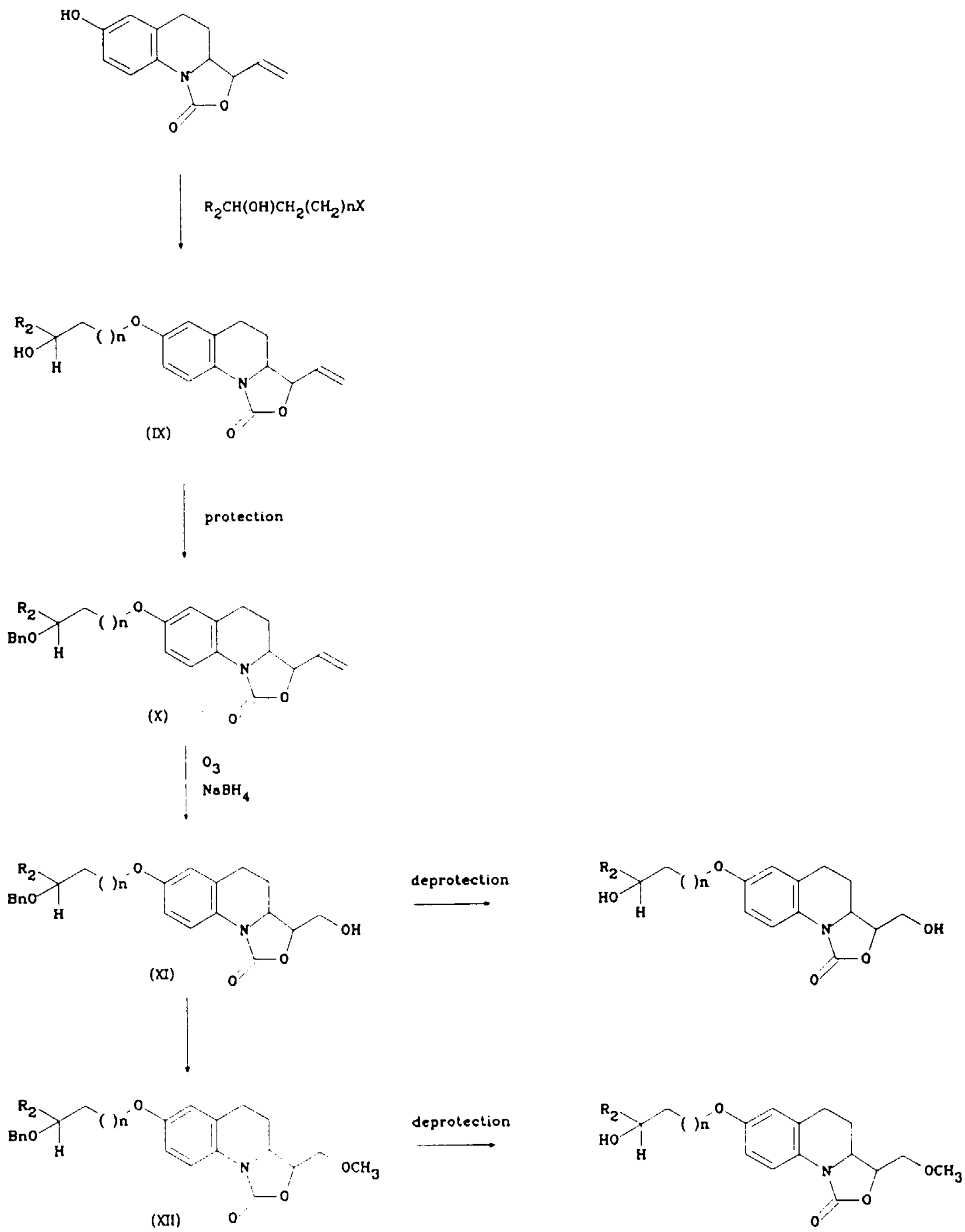


(IX)

(X)

(XI)

(XII)

## Annexe 3

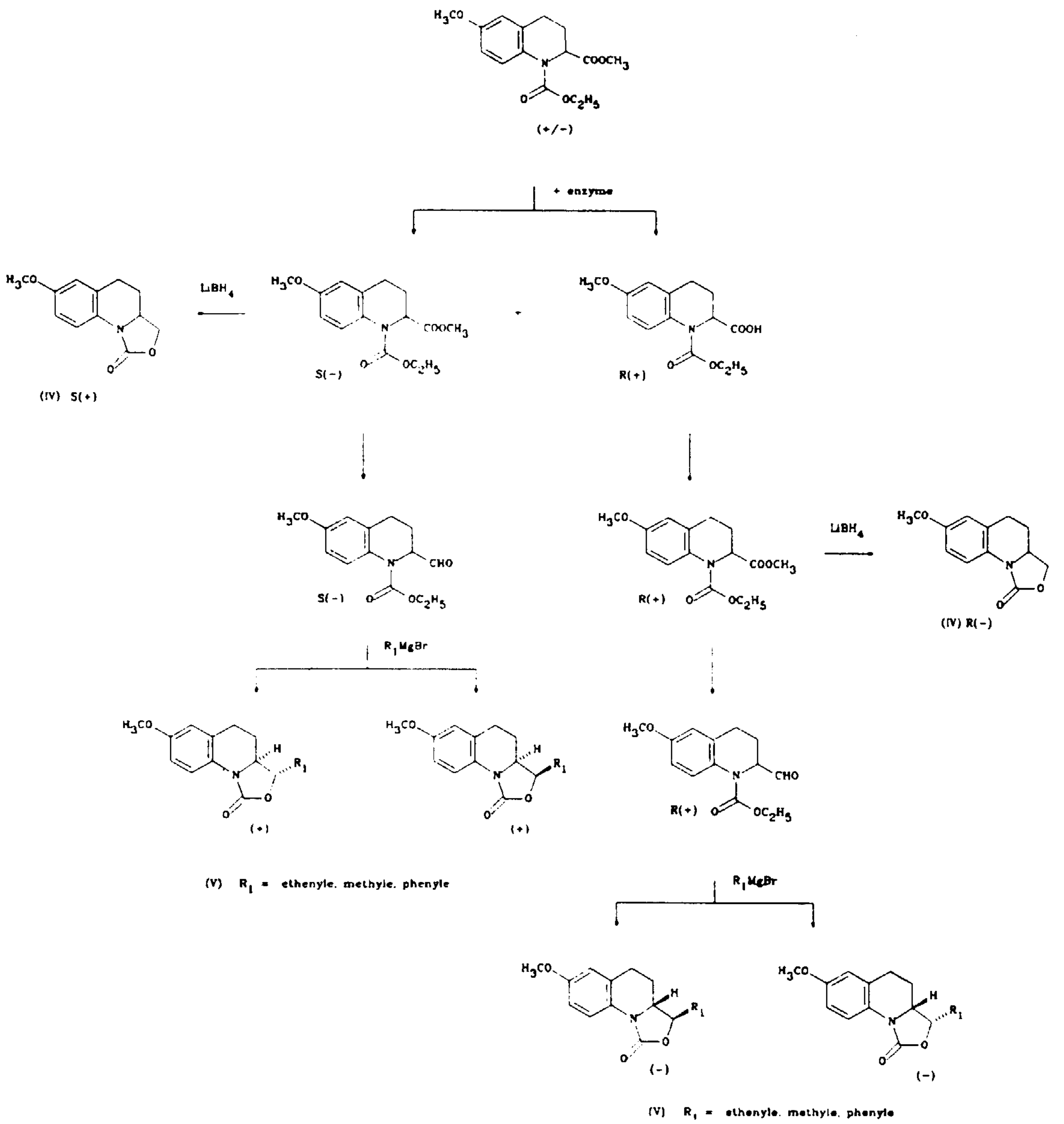


## Revendications

**1.** Dérivés de 3,3*a*,4,5-tétrahydro-1*H*-oxazolo[3,4-*a*]quinoléin-1-one de formule générale (I)

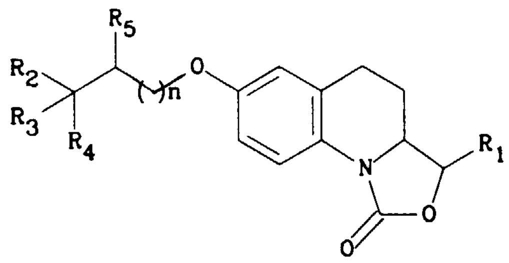

(I)

dans laquelle :

n est égal à 0 ou 1,
$R_1$ représente un atome d'hydrogène ou un groupe éthényle, méthyle, éthyle, phényle, hydroxyméthyle ou méthoxyméthyle, et,
soit $R_2$ est un groupe méthyle, trifluorométhyle ou cyano, $R_3$ est un atome d'hydrogène ou un groupe hydroxyle ou benzyloxy et $R_4$ et $R_5$ sont des atomes d'hydrogène et si $R_2$ est un méthyle $R_3$ n'est pas un hydrogène,
soit $R_2$ et $R_4$ forment ensemble un groupe $-(CH_2)_4-$, $R_3$ est un groupe hydroxyle et $R_5$ est un atome d'hydrogène,
soit $R_2$ et $R_5$ forment ensemble un groupe $-O-(CH_2)_3-$et $R_3$ et $R_4$ sont des atomes d'hydrogène,
soit $R_2$ et $R_5$ forment ensemble un groupe $-(CH_2)_4-$, $R_3$ est un groupe hydroxyle et R4 est un atome d'hydrogène, sous forme d'énantiomères et de diastéréoisomères et de mélanges de ces différentes formes, y compris de mélanges racémiques.

**2.** Composés de formule (I) selon la revendication 1, à savoir la 3-méthoxyméthyl-7-(4,4,4-trifluoro-3-hydroxybutoxy)-3,3*a*,4,5-tétrahydro-1*H*-oxazolo[3,4-*a*]quinoléin-1-one, ses énantiomères et ses diastéréoisomères purs ou sous forme de mélanges.

**3.** Composés de formule (I) selon la revendication 1, à savoir la 3-méthoxyméthyl-7-(4,4,4-trifluorobutoxy)-3,3*a*,4,5-tétrahydro-1*H*-oxazolo[3,4-*a*]quinoléin-1-one, ses énantiomères et ses diastéréoisomères purs ou sous forme de mélanges.

**4.** Composés de formule (I) selon la revendication 1, à savoir la 7-(4,4,4-trifluorobutoxy)-3,3*a*,4,5-tétrahydro-1*H*-oxazolo[3,4-*a*]quinoléin-1-one et ses énantiomères purs ou sous forme de mélanges.

**5.** Composés de formule (I) selon la revendication 1, à savoir la 7-(3-hydroxy-4,4,4-trifluorobutoxy)-3,3*a*,4,5-tétrahydro-1*H*-oxazolo[3,4-*a*]quinoléin-1-one, ses énantiomères et ses diastéréoisomères purs ou sous forme de mélanges.

**6.** Composés de formule (I) selon la revendication 1, à savoir la 3-méthoxyméthyl-7-[(2-(1-hydroxycyclopentyl) éthoxy]-3,3*a*,4,5-tétrahydro-1*H*-oxazolo[3,4-*a*]quinoléin-1-one, ses énantiomères et ses diastéréoisomères purs ou sous forme de mélanges.

**7.** Procédé de préparation des composés de formule (I) selon la revendication 1, caractérisé en ce que l'on traite un composé de formule (II)

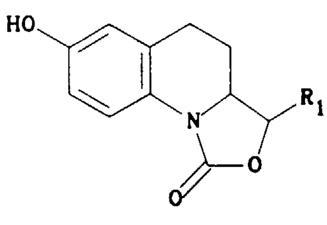

(II)

dans laquelle $R_1$ représente un atome d'hydrogène, un groupe éthényle, méthyle, phényle, hydroxyméthyle ou méthoxyméthyle, par un composé de formule (III)

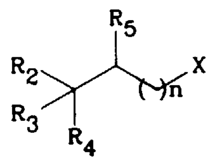

(III)

dans laquelle $R_2$, $R_3$, $R_4$, $R_5$ et n sont définis comme dans la revendication 1 et X est un atome d'halogène ou un groupe labile tel que mésyloxy ou tosyloxy, pour obtenir un composé de formule (I) dans laquelle $R_1$ est défini comme précédemment, puis à réduire le composé de formule (I) dans laquelle $R_1$ est un groupe éthényle, pour obtenir le composé de formule (I) dans laquelle $R_1$ est un groupe éthyle.

**8.** Procédé, selon la revendication 7, pour la préparation des énantiomères et diastéréoisomères des composés de formule (I), caractérisé en ce que l'on utilise comme produits de départ, les isomères correspondants des composé de formule (II).

**9.** Procédé de préparation selon la revendication 8, caractérisé en ce que les énantiomères et diastéréoisomères des composés de formule (II) sont préparés à partir des énantiomères ou diastéréoisomères des composés de formule (IV)

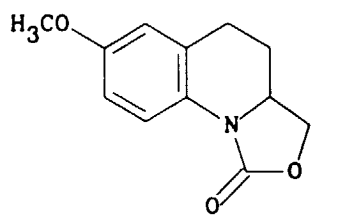

(IV)

ou (V)

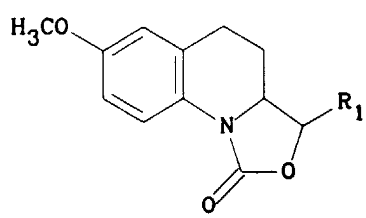

(V)

dans laquelle $R_1$ représente un groupe éthényle, méthyle ou phényle,
obtenus par hydrolyse enzymatique du 2-méthoxycarbonyl-6-méthoxy-1,2,3,4-tétrahydroquinoléine-1-carboxylate d'éthyle, séparation par extraction, de l'énantiomère S(-) du 2-méthoxy carbonyl-6-méthoxy-1,2,3,4-tétrahydroquinoléine-1-carboxylate d'éthyle et de l'énantiomère R(+) du 2-carboxy-6-méthoxy-1,2,3,4-tétrahydroquinoléine-1-carboxylate d'éthyle, traitement par le chlorure de thionyle et le méthanol de l'énantiomère R(+) du 2-carboxy-6-méthoxy-1,2,3,4-tétrahydro quinoléine-1-carboxylate d'éthyle conduisant au dérivé 2-méthoxycarbonyl correspondant, et réaction des énantiomères S(-) et R(+) du dérivé 2-méthoxycarbonyl, soit avec le borohydrure de lithium, pour obtenir respectivement les énantiomères S(+) et R(-) du composé de formule (IV), soit avec l'hydrure de diisobutylaluminium pour obtenir respectivement les énantiomères S(-) et R(+) du dérivé 2-formyl correspondant, qui, par traitement par un organomagnésien de formule $R_1MgX$ dans laquelle $R_1$ est défini comme précédemment et X est un atome d'halogène, puis par le méthanolate de sodium et enfin séparation par chromatographie, conduisent aux diastéréoisomères des composé de formule (V) .

**10.** Procédé selon la revendication 9, pour la préparation des énantiomères du 2-méthoxycarbonyl-6-méthoxy-1,2,3,4-tétra hydroquinoléine-1-carboxylate d'éthyle caractérisé en ce que l'hydrolyse enzymatique est réalisée par des extraits enzymatiques tels que l'estérase de foie de porc ou les poudres acétoniques de foie de cheval, de porc, de boeuf, de lapin ou de mouton.

**11.** Médicament caractérisé en ce qu'il consiste en un composé de formule (I) selon la revendication 1.

**12.** Composition pharmaceutique caractérisée en ce qu'elle contient un composé de formule (I) selon la revendication 1 en association avec tout excipient approprié.

**Patentansprüche**

**1.** 3,3*a*,4,5-Tetrahydro-1*H*-oxazolo[3,4-*a*]chinolin-1-on-Derivate der allgemeinen Formel (I)

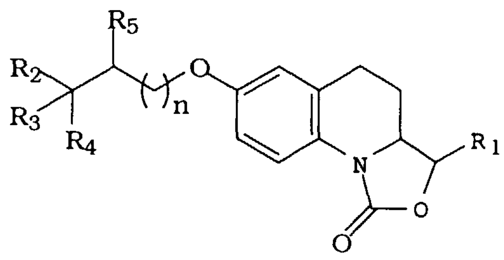

(I)

in der:

n 0 oder ist ist,
$R_1$ ein Wasserstoffatom oder eine Ethenyl-, Methyl-, Ethyl-, Phenyl-, Hydroxymethyl- oder Methoxymethylgruppe bedeutet und
entweder $R_2$ eine Methyl-, Trifluormethyl- oder Cyanogruppe, $R_3$ ein Wasserstoffatom oder eine Hydroxy- oder Benzyloxygruppe und $R_4$ und $R_5$ Wasserstoffatome bedeuten und, wenn $R_2$ eine Methylgruppe darstellt, $R_3$ kein Wasserstoffatom bedeutet,
oder $R_2$ und $R_4$ gemeinsam eine Gruppe $-(CH_2)_4-$, $R_3$ eine Hydroxygruppe und $R_5$ ein Wasserstoffatom bedeuten,
oder $R_2$ und $R_5$ gemeinsam eine Gruppe $-O-(CH_2)_3-$ und $R_3$ und $R_4$ Wasserstoffatome bedeuten,
oder $R_2$ und $R_5$ gemeinsam eine Gruppe $-(CH_2)_4-$, $R_3$ eine Hydroxygruppe und $R_4$ ein Wasserstoffatom bedeuten,
in Form der Enantiomeren und der Diastereoisomeren und von Mischungen dieser verschiedenen Formen einschließlich racemischer Mischungen.

**2.** Verbindungen der Formel (I) nach Anspruch 1, nämlich 3-Methoxymethyl-7-(4,4,4-trifluor-3-hydroxybutoxy)-3,3a, 4,5-tetrahydro-1*H*-oxazolo[3,4-*a*]chinolin-1-on, dessen Enantiomere und dessen Diastereoisomere in reiner Form oder in Form von Mischungen.

**3.** Verbindungen der Formel (I) nach Anspruch 1, nämlich 3-Methoxymethyl-7-[4,4,4-trifluorbutoxy)-3,3a,4,5-tetrahydro-1*H*-oxazolo[3,4-*a*]chinolin-1-on, dessen Enantiomere und dessen Diastereoisomere in reiner Form oder in Form von Mischungen.

**4.** Verbindungen der Formel (I) nach Anspruch 1, nämlich 7-(4,4,4-Trifluorbutoxy)-3,3a,4,5-tetrahydro-1*H*-oxazolo [3,4-*a*]chinolin-1-on und dessen Enantiomere in reiner Form oder in Form von Mischungen.

**5.** Verbindungen der Formel (I) nach Anspruch 1, nämlich 7-(3-Hydroxy-4,4,4-trifluorbutoxy)-3,3a,4,5-tetrahydro-1*H*-oxazolo[3,4-*a*]chinolin-1-on, dessen Enantiomere und dessen Diastereoisomere in reiner Form oder in Form von Mischungen.

**6.** Verbindungen der Formel (I) nach Anspruch 1, nämlich 3-Methoxymethyl-7-[(2-(1-hydroxycyclopentyl)-ethoxy]-3,3a,4,5-tetrahydro-1*H*-oxazolo[3,4-*a*]chinolin-1-on, dessen Enantiomere und dessen Diastereoisomere in reiner Form oder in Form von Mischungen.

**7.** Verfahren zur Herstellung der Verbindungen der Formel (I) nach Anspruch 1, dadurch gekennzeichnet, daß man eine Verbindung der Formel (II)

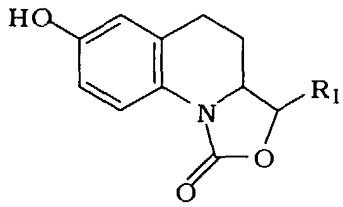

(II)

in der $R_1$ ein Wasserstoffatom, eine Ethenyl-, Methyl-, Phenyl-, Hydroxymethyl- oder Methoxymethylgruppe bedeutet,
mit einer Verbindung der Formel (III)

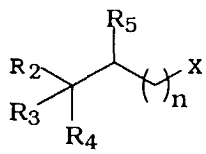

(III)

in der $R_2$, $R_3$, $R_4$, $R_5$ und n die in Anspruch 1 angegebenen Bedeutungen besitzen und X ein Halogenatom oder eine labile Gruppe, wie eine Mesyloxygruppe oder eine Tosyloxygruppe darstellt, umsetzt zur Bildung einer Verbindung der Formel (I), in der $R_1$ die oben angegebenen Bedeutungen besitzt, wonach man die Verbindung der Formel (I), in der $R_1$ eine Ethenylgruppe darstellt, reduziert zur Bildung der Verbindung der Formel (I), in der $R_1$ eine Ethylgruppe bedeutet.

**8.** Verfahren nach Anspruch 7 zur Herstellung der Enantiomeren und Diastereoisomeren der Verbindungen der Formel (I), dadurch gekennzeichnet, daß man als Ausgangsprodukte die entsprechenden Isomeren der Verbindungen der Formel (II) verwendet.

**9.** Herstellungsverfahren nach Anspruch 8, dadurch gekennzeichnet, daß man die Enantiomeren oder Diastereoisomeren der Verbindungen der Formel (II) herstellt ausgehend von den Enantiomeren oder Diastereoisomeren der Verbindungen der Formel (IV)

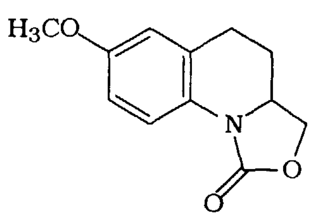

(IV)

oder (V)

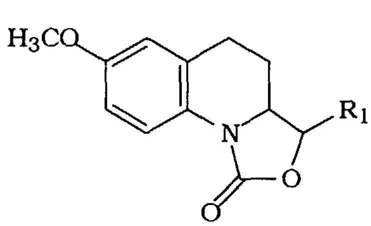

(V)

worin $R_1$ eine Ethenyl-, Methyl- oder Phenylgruppe bedeutet,
die man durch enzymatische Hydrolyse von 2-Methoxycarbonyl-6-methoxy-1,2,3,4-tetrahydrochinolin-1-carbonsäureethylester, Trennung durch Extraktion des S(-)-Enantiomeren des 2-Methoxycarbonyl-6-methoxy-1,2,3,4-tetrahydrochinolin-1-carbonsäureethylesters und des R(+)-Enantiomeren des 2-Carboxy-6-methoxy-1,2,3,4-tetrahydrochinolin-1-carbonsäureethylesters, Behandlung des R(+)-Enantiomeren des 2-Carboxy-6-methoxy-1,2,3,4-tetrahydrochinolin-1-carbonsäureethylesters mit Thionylchlorid und Methanol zur Bildung des entsprechenden 2-Methoxycarbonyl-Derivats und Umsetzung der S(-)- und R(+)-Enantiomeren des 2-Methoxycarbonyl-Derivats entweder mit Lithiumborhydrid zur Bildung des S(+)- bzw. R(-)-Enantiomeren der Verbindung der Formel (IV), oder mit Diisobutylaluminiumhydrid zur Bildung des S(-)- bzw. R(+)-Enantiomeren des entsprechenden 2-Formylderivats, welche durch Behandeln mit einer magnesiumorganischen Verbindung der Formel $R_1MgX$, worin $R_1$ die oben angegebenen Bedeutungen besitzt und X ein Halogenatom darstellt, und dann mit Natriummethanolat und schließlich durch chromatographische Trennung zu den Diastereoisomeren der Verbindungen der Formel (V) führen.

**10.** Verfahren nach Anspruch 9 zur Herstellung der Enantiomeren des 2-Methoxycarbonyl-6-methoxy-1,2,3,4-tetrahydrochinolin-1-carbonsäureethylesters, dadurch gekennzeichnet, daß die enzymatische Hydrolyse mit enzymatischen Extrakten, wie Schweineleberesterase oder Acetonpulvern von Lebern von Pferden, Schweinen, Rindern, Kaninchen oder Schafen durchgeführt wird.

**11.** Arzneimittel, dadurch gekennzeichnet, daß es aus einer Verbindung der Formel (I) nach Anspruch 1 besteht.

**12.** Pharmazeutische Zubereitung, dadurch gekennzeichnet, daß sie eine Verbindung der Formel (I) nach Anspruch 1 in Kombination mit irgendeinem geeigneten Trägermaterial enthält.

## Claims

**1.** 3,3a,4,5-Tetrahydro-1H-oxazolo[3,4-a]quinolin-1-one derivatives of general formula (I)

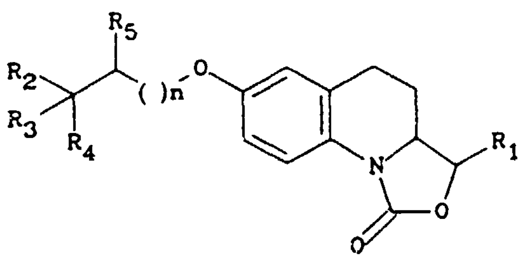

(I)

in which:

n is equal to 0 or 1,
$R_1$ represents a hydrogen atom or an ethenyl, methyl, ethyl, phenyl, hydroxymethyl or methoxymethyl group, and
either $R_2$ is a methyl, trifluoromethyl or cyano group, $R_3$ is a hydrogen atom or a hydroxyl or benzyloxy group and $R_4$ and $R_5$ are hydrogen atoms and if $R_2$ is a methyl $R_3$ is not a hydrogen,
or $R_2$ and $R_4$ together form a $-(CH_2)_4-$ group, $R_3$ is a hydroxyl group and $R_5$ is a hydrogen atom,
or $R_2$ and $R_5$ together form an $-O-(CH_2)_3-$ group, and $R_3$ and $R_4$ are hydrogen atoms,
or $R_2$ and $R_5$ together form a $-(CH_2)_4-$ group, $R_3$ is a hydroxyl group and $R_4$ is a hydrogen atom, in the form of enantiomers and of diastereoisomers and of mixtures of these various forms, including racemic mixtures.

**2.** Compounds of formula (I) according to Claim 1, namely 3-methoxymethyl-7-(4,4,4-trifluoro-3-hydroxybutoxy)-3,3a,4,5-tetrahydro-1H-oxazolo[3,4-a]quinolin-1-one, and the enantiomers and diastereoisomers thereof which are pure or in the form of mixtures.

**3.** Compounds of formula (I) according to Claim 1, namely 3-methoxymethyl-7-(4,4,4-trifluorobutoxy)-3,3a,4,5-tetrahydro-1H-oxazolo[3,4-a]quinolin-1-one, and the enantiomers and diastereoisomers thereof which are pure or in the form of mixtures.

**4.** Compounds of formula (I) according to Claim 1, namely 7-(4,4,4-trifluorobutoxy)-3,3a,4,5-tetrahydro-1H-oxazolo[3,4-a]quinolin-1-one, and the enantiomers thereof which are pure or in the form of mixtures.

**5.** Compounds of formula (I) according to Claim 1, namely 7-(3-hydroxy-4,4,4-trifluorobutoxy)-3,3a,4,5-tetrahydro-1H-oxazolo[3,4-a]quinolin-1-one, and the enantiomers and diastereoisomers thereof which are pure or in the form of mixtures.

**6.** Compounds of formula (I) according to Claim 1, namely 3-methoxymethyl-7-[(2-(1-hydroxycyclopentyl)ethoxy]-3,3a,4,5-tetrahydro-1H-oxazolo[3,4-a]quinolin-1-one, and the enantiomers and diastereoisomers thereof which are pure or in the form of mixtures.

**7.** Process for the preparation of the compounds of formula (I) according to Claim 1, characterized in that a compound of formula (II)

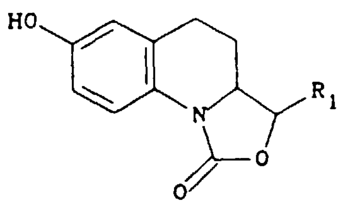

(II)

in which $R_1$ represents a hydrogen atom or an ethenyl, methyl, phenyl, hydroxymethyl or methoxymethyl group, is treated with a compound of formula (III)

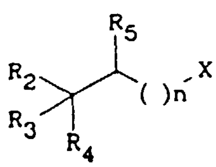

(III)

in which $R_2$, $R_3$, $R_4$, Rs and n are defined as in Claim 1 and X is a halogen atom or a labile group such as mesyloxy or tosyloxy, in order to obtain a compound of formula (I) in which $R_1$ is defined as above, and then, in reducing the compound of formula (I) in which $R_1$ is an ethenyl group, in order to obtain the compound of formula (I) in which $R_1$ is an ethyl group.

**8.** Process according to Claim 7 for the preparation of the enantiomers and diastereoisomers of the compounds of formula (I), characterized in that the corresponding isomers of the compounds of formula (II) are used as starting materials.

**9.** Preparation process according to Claim 8, characterized in that the enantiomers and diastereoisomers of the compounds of formula (II) are prepared from the enantiomers or diastereoisomers of che compounds of formula (IV)

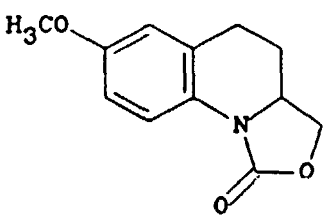

(IV)

or (V)

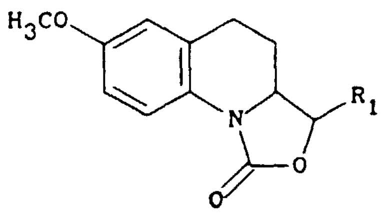

(V)

in which $R_1$ represents an ethenyl, methyl or phenyl group, which are obtained by enzymatic hydrolysis of ethyl 2-methoxycarbonyl-6-methoxy-1,2,3,4-tetrahydroquinoline-1-carboxylate, separation by extraction of the S(-) enantiomer of ethyl 2-methoxycarbonyl-6-methoxy-1,2,3,4-tetrahydroquinoline-1-carboxylate and of the R(+) enantiomer of ethyl 2-carboxy-6-methoxy-1,2,3,4-tetrahydroquinoline-1-carboxylate, treatment with thionyl chloride and methanol of the R(+) enantiomer of ethyl 2-carboxy-6-methoxy-1,2,3,4-tetrahydroquinoline-1-carboxylate leading to the corresponding 2-methoxycarbonyl derivative, and reaction of the S(-) and R(+) enantiomers of the 2-methoxycarbonyl derivative, either with lithium borohydride, in order to obtain the S(+) and R(-) enantiomers respectively of the compound of formula (IV), or with diisobutylaluminium hydride, in order to obtain the S(-) and R(+) enantiomers respectively of the corresponding 2-formyl derivative, which, on treatment with an organomagnesium compound of formula RiMgX, in which $R_1$ is defined as above and X is a halogen atom, and then with sodium methoxide and finally separation by chromatography, lead to the diastereoisomers of the compounds of formula (V).

**10.** Process according to Claim 9 for the preparation of the enantiomers of ethyl 2-methoxycarbonyl-6-methoxy-1,2,3,4-tetrahydroquinoline-1-carboxylate,
characterized in that the enzymatic hydrolysis is performed by enzyme extracts such as pig liver esterase or horse, pig, bovine, rabbit or sheep liver acetone powders.

**11.** Drug, characterized in that it consists of a compound of formula (I) according to Claim 1.

**12.** Pharmaceutical composition, characterized in that it contains a compound of formula (I) according to Claim 1, in combination with any suitable excipient.